# EUROPEAN PATENT APPLICATION

(11) **EP 4 060 036 A1**
(43) Date of publication of application: **21.09.2022**
(21) Application number: 21162531.4
(22) Date of filing: 15.03.2021
(51) Int. Cl.: C12N 9/22, C11D 3/386, C12N 15/52, C11D 11/00

(54) **POLYPEPTIDE VARIANTS**

(71) Applicant: Novozymes A/S, 2880 Bagsvaerd (DK)
(72) Inventor: MALTEN, Marco, 2880 Bagsvaerd (DK); JOHANSEN, Annette, Helle, 2880 Bagsvaerd (DK); OESTERGAARD, Lars, Henrik,, 2880 Bagsvaerd (DK); CHRISTENSEN, Lars, Lehmann, Hylling, 2880 Bagsvaerd (DK); OEHLENSCHLAEGER, Christian, Berg,, 2880 Bagsvaerd (DK)
(74) Representative: NZ EPO Representatives

(57) **Abstract**

The present invention relates to polypeptide variants having DNase activity, as well as detergent compositions comprising the variants, use of the variants for cleaning, and methods for obtaining the variants.

## Description

### Reference to a Sequence Listing

This application contains a Sequence Listing in computer readable form, which is incorporated herein by reference.

### Background of the Invention

### Field of the Invention

The present invention relates to novel DNase variants exhibiting improved properties such as detergent stability and/or storage stability. The variants of the invention are suitable for use in cleaning processes and detergent compositions, such as laundry compositions and dishwashing compositions, including hand dishwashing and automatic dishwashing compositions. The invention also relates to isolated DNA sequences encoding the variants, expression vectors, host cells, and methods for producing and using the DNase variants of the invention.

### Description of the Related Art

Enzymes have been used in detergents for decades, with the most commercially relevant being proteases and amylases to effectively remove protein and starch related soiling from laundry, dishes, etc. However, most household care-related soiling is a complex mixture of various organic materials. Materials such as fabrics and hard surfaces are exposed to microorganisms from the environment as well as from e.g. the body in the case of clothes and other fabrics. Further, build-up of organic material is a source for adhesion and multiplication of microorganisms, which may adhere to soil on laundry or other surfaces, where such adhered microorganisms may propagate and form biofilm. Much biofilm is embedded in a self-produced matrix of extracellular polymeric substance (EPS). Biofilm EPS is a polymeric conglomeration generally composed of extracellular DNA, proteins, and polysaccharides. Household soiling includes different organic materials including not only protein, starch and oil, but also e.g. DNA.

Extracellular DNA (eDNA) is known to be a key component of biofilms. It has been shown that eDNA together with protein provides structural integrity to bacterial biofilm, and that treatment with DNasel results in change in biofilm structure leading to loss of biofilm material (Blakeman et al., Langmuir 2019, 35, 6468-6475).

Detergent compositions containing DNases are known from the patent literature, while on the other hand there are relatively few commercially available cleaning products containing a DNase. One reason for this may be that to be useful in cleaning processes such as laundry, an enzyme such as a DNase needs to be stable in detergent compositions and compatible with standard detergent components such as surfactants, builders, bleaches etc.

The present invention provides DNase enzymes which are suitable for use in detergents for e.g. laundry or dishwashing, and which have improved stability in detergent compositions.

### Summary of the Invention

The present invention provides polypeptides having DNase activity and which have improved stability in detergent compositions. The polypeptides of the invention are variants of SEQ ID NO: 1 comprising at least one substitution, and typically two or more substitutions, selected from the group consisting of N61D, T65I, T65V, S82R, K107Q, T127S, T127V, G149N, S164D and L181S. The invention also provides methods for obtaining such variants as well as isolated polynucleotides encoding the DNase variants of the invention, nucleic acid constructs and expression vectors capable of expressing the polynucleotides; and host cells comprising said polynucleotide.

### Sequences

SEQ ID NO: 1 variant of the polypeptide of SEQ ID NO: 27 from *Bacillus cibi*
SEQ ID NO: 2 mature polypeptide obtained from *Bacillus horikoshii*
SEQ ID NO: 3 mature polypeptide obtained from *Bacillus sp-62520*
SEQ ID NO: 4 mature polypeptide obtained from *Bacillus horikoshii*
SEQ ID NO: 5 mature polypeptide obtained from *Bacillus horikoshii*
SEQ ID NO: 6 mature polypeptide obtained from *Bacillus sp-16840*
SEQ ID NO: 7 mature polypeptide obtained from *Bacillus sp-16840*
SEQ ID NO: 8 mature polypeptide obtained from *Bacillus sp-62668*
SEQ ID NO: 9 mature polypeptide obtained from *Bacillus sp-13395*
SEQ ID NO: 10 mature polypeptide obtained from *Bacillus horneckiae*
SEQ ID NO: 11 mature polypeptide obtained from *Bacillus sp-11238*
SEQ ID NO: 12 mature polypeptide obtained from *Bacillus sp-62451*
SEQ ID NO: 13 mature polypeptide obtained from *Bacillus sp-18318*
SEQ ID NO: 14 mature polypeptide obtained from *Bacillus idriensis*
SEQ ID NO: 15 is the mature polypeptide obtained from *Bacillus algicola*
SEQ ID NO: 16 mature polypeptide obtained from *environmental sample J*
SEQ ID NO: 17 mature polypeptide obtained from *Bacillus vietnamensis*
SEQ ID NO: 18 mature polypeptide obtained from *Bacillus hwajinpoensis*
SEQ ID NO: 19 mature polypeptide obtained from *Paenibacillus mucilaginosus*
SEQ ID NO: 20 mature polypeptide obtained from *Bacillus indicus*
SEQ ID NO: 21 mature polypeptide obtained from *Bacillus marisflavi*
SEQ ID NO: 22 mature polypeptide obtained from *Bacillus luciferensis*
SEQ ID NO: 23 mature polypeptide obtained from *Bacillus marisflavi*
SEQ ID NO: 24 mature polypeptide obtained from *Bacillus sp. SA2-6*
SEQ ID NO: 25 motif [D/M/L][S/T]GYSR[D/N]
SEQ ID NO: 26 motif ASXNRSKG
SEQ ID NO: 27 mature polypeptide obtained from *Bacillus cibi*
SEQ ID NO: 28 protease variant

### Definitions

The term "adjunct material" or "adjunct ingredient" means any liquid, solid or gaseous material selected for the particular type of detergent composition desired and the form of the product (e.g., liquid, granule, powder, bar, paste, spray, tablet, gel, or foam composition), which materials are also preferably compatible with the DNase variant enzyme used in the composition.

The term "biofilm" means any group of microorganisms in which cells stick to each other on a surface, such as a textile, dishware or a hard surface. These adherent cells are frequently embedded within a self-produced matrix of extracellular polymeric substance (EPS). Biofilm EPS is a polymeric conglomeration generally composed of extracellular DNA, proteins and polysaccharides. Biofilms may form on living or non-living surfaces. The microbial cells growing in a biofilm are physiologically distinct from planktonic cells of the same organism, which, by contrast, are single-cells that may float or swim in a liquid medium. Bacteria living in a biofilm usually have significantly different properties from free-floating bacteria of the same species, as the dense and protected environment of the film allows them to cooperate and interact in various ways. One benefit of this environment for the bacteria is increased resistance to detergents and antibiotics, as the dense extracellular matrix and the outer layer of cells protect the interior of the bacterial community. On laundry, biofilm-producing bacteria can be found e.g. among the following species: *Acinetobactersp., Aeromicrobium* sp., *Brevundimonas* sp., *Microbacterium* sp., *Micrococcus luteus, Pseudomonas* sp., *Staphylococcus epidermidis* and *Stenotrophomonas* sp.

The term "clade" means a group of polypeptides clustered together based on homologous features traced to a common ancestor. Polypeptide clades can be visualized as phylogenetic trees, and a clade is a group of polypeptides that consists of a common ancestor and all of its lineal descendants. Polypeptides forming a group, e.g. a clade, as shown in a phylogenetic tree often share common properties and are more closely related than other polypeptides not belonging to the clade.

The term "cleaning composition" or "detergent composition" means any composition adapted for cleaning of e.g. laundry, dishware, or hard surfaces such as floors, tables, walls etc. It includes, unless otherwise indicated, granular or powder-form all-purpose or heavy-duty washing agents, especially cleaning detergents; liquid, gel or paste-form all-purpose washing agents, especially the so- called heavy-duty liquid (HDL) types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, cleaning bars, soap bars, mouthwashes, denture cleaners, car or carpet shampoos, bathroom cleaners; hair shampoos and hair-rinses; shower gels, foam baths; metal cleaners; as well as cleaning auxiliaries such as bleach additives and "stain-stick" or pre-treat types. The terms "cleaning composition" and "cleaning formulation" are used in reference to mixtures which are intended for use in a wash medium for the cleaning of soiled objects. In some aspects, the term is used in reference to laundering fabrics and/or garments (e.g., "laundry detergents"). In alternative aspects, the term refers to other detergents, such as those used to clean dishes, cutlery, etc. (e.g., "dishwashing detergents"). It is not intended that the present invention be limited to any particular detergent formulation or composition such as compositions that contain surfactants. In addition to the DNase variants according to the invention, detergent compositions may contain, e.g., surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anti-corrosion agents, enzyme inhibitors or stabilizers, enzyme activators, transferase(s), hydrolytic enzymes, oxido reductases, bluing agents and fluorescent dyes, antioxidants, and solubilizers.

The term "deep cleaning" refers to disruption or removal of components of organic matter, e.g. biofilm, such as polysaccharides, proteins, DNA, soil or other components present in the organic matter.

The terms "DNase", "DNase variant" and "DNase parent" refer to a polypeptide with DNase activity (i.e. deoxyribonuclease activity) that catalyzes the hydrolytic cleavage of phosphodiester linkages in DNA, thus degrading DNA. DNases belong to the esterases (EC-number 3.1), a subgroup of the hydrolases. The DNases are classified e.g. in E.C. 3.1.11, E.C. 3.1.12, E.C. 3.1.15, E.C. 3.1.16, E.C. 3.1.21, E.C 3.1.22, E.C 3.1.23, E.C 3.1.24 and E.C.3.1.25 as well as EC 3.1.21.X, where X=1, 2, 3, 4, 5, 6, 7, 8 or 9. The term "DNase" and the expression "a polypeptide with DNase activity" or "DNase polypeptide" may be used interchangeably throughout the application. For purposes of the present invention, DNase activity may be determined according to the procedure described in Assay I below. The DNase variants of the present invention preferably have at least one improved property compared to the parent DNase. In one embodiment, the DNase variants have improved stability, e.g. improved storage stability in a detergent composition, compared to the parent DNase. Preferably, DNase variants of the invention have a half-life improvement factor (T½ IF, or HIF) of at least 1.1, more preferably at least 1.2, preferably at least 1.5, more preferably at least 2, such as at least 3, after storage in a liquid detergent, determined e.g. as described in Example 3 herein, relative to the half-life of a reference DNase, for example the DNase of SEQ ID NO: 1. The half-life improvement factor of a DNase variant of the invention is calculated as the half-life of a DNase variant relative to the half-life of the reference DNase.

In another embodiment, the DNase variants may have improved DNase activity compared to the parent DNase.

The term "effective amount" in relation to an enzyme refers to the quantity of enzyme necessary to achieve the enzymatic activity required in the specific application, e.g., in a defined detergent composition. Such effective amounts are readily ascertained by one of ordinary skill in the art and are based on many factors, such as the particular enzyme used, the cleaning application, the specific composition of the detergent composition, and whether a liquid or dry (e.g., granular, bar) composition is required, and the like. The term "effective amount" of a DNase variant refers to the quantity of DNase variant described hereinbefore that achieves a desired level of enzymatic activity, e.g., in a defined detergent composition.

The term "expression" includes any step involved in the production of the variant including, but not limited to, transcription, post-transcriptional modification, translation, post-translational modification, and secretion.

The term "expression vector" means a linear or circular DNA molecule that comprises a polynucleotide encoding a variant and is operably linked to additional nucleotides that provide for its expression.

The term "fabric" encompasses any textile material. Thus, it is intended that the term encompass garments, as well as fabrics, yarns, fibers, non-woven materials, natural materials, synthetic materials, and any other textile material.

The term "high detergent concentration" system includes detergents where greater than about 2000 ppm of detergent components is present in the wash water. European detergents are generally considered to be high detergent concentration systems.

The term "medium detergent concentration" system includes detergents where between about 800 ppm and about 2000 ppm of detergent components is present in the wash water. North American detergents are generally considered to be medium detergent concentration systems.

The term "low detergent concentration" system includes detergents where less than about 800 ppm of detergent components is present in the wash water. Asian, e.g., Japanese detergents are typically considered low detergent concentration systems.

The term "host cell" means any cell type that is susceptible to transformation, transfection, transduction, and the like with a nucleic acid construct or expression vector comprising a polynucleotide of the present invention. The term "host cell" encompasses any progeny of a parent cell that is not identical to the parent cell due to mutations that occur during replication.

The term "improved property" means a characteristic associated with a variant that is improved compared to the parent and/or compared to the DNase polypeptide having SEQ ID NO: 1, or compared to a DNase having the identical amino acid sequence of said variant but not having the alterations defined herein at one or more of said specified positions. Such improved properties include, but are not limited to, stability, such as detergent stability, e.g. detergent storage stability, and wash performance, e.g. deep cleaning effect, where the deep-cleaning effect may include a de-gluing effect. The DNase variants of the invention preferably have improved stability, in particular improved detergent storage stability, compared to the polypeptide of SEQ ID NO: 1.

The term "improved DNase activity" is defined herein as an altered DNase activity e.g. by increased hydrolytic cleavage of phosphodiester linkages in the DNA by a DNase variant of the invention relative to the activity of the parent DNase, such as compared to a DNase with SEQ ID NO: 1.

The term "improved wash performance" includes but is not limited to the term "deep cleaning effect". Improved performance e.g. deep cleaning performance of a DNase variant according to the invention is measured compared to the DNase parent, e.g. the DNase shown in SEQ ID NO: 1 or SEQ ID NO: 27. The improved performance e.g. deep cleaning performance may be expressed as a Remission value of the stained swatches. After washing and rinsing the swatches are spread out flat and allowed to air dry at room temperature overnight. All washed swatches are evaluated the day after washing. Light reflectance evaluations of the swatches are performed using a Macbeth Color Eye 7000 reflectance spectrophotometer with a very small aperture. The measurements are made without UV in the incident light and remission at 460 nm is extracted. A positive response indicates that soil has been removed; this may include soiled that sticks to the fabric due to e.g. a sticky biofilm layer.

The term "mature polypeptide" means a polypeptide in its final form following translation and any post-translational modifications, such as N-terminal processing, C-terminal truncation, glycosylation, phosphorylation, etc. The N-terminals of a mature polypeptide may be experimentally determined based on EDMAN N-terminal sequencing data and Intact MS data. It is known in the art that a host cell may produce a mixture of two of more different mature polypeptides (*i.e.,* with a different C-terminal and/or N-terminal amino acid) expressed by the same polynucleotide.

The term "mature polypeptide coding sequence" means a polynucleotide that encodes a mature polypeptide having DNase activity.

The term "nucleic acid construct" means a nucleic acid molecule, either single- or doublestranded, which is isolated from a naturally occurring gene or is modified to contain segments of nucleic acids in a manner that would not otherwise exist in nature or which is synthetic. The term nucleic acid construct is synonymous with the term "expression cassette" when the nucleic acid construct contains the control sequences required for expression of a coding sequence of the present invention.

The term "operably linked" means a configuration in which a control sequence is placed at an appropriate position relative to the coding sequence of a polynucleotide such that the control sequence directs the expression of the coding sequence.

The terms "parent DNase", "DNase parent" or "precursor DNase" may be used interchangeably. In the context of the present invention, a "parent DNase" is to be understood as a DNase into which at least one alteration is made in the amino acid sequence to produce a DNase variant having an amino acid sequence which is less than 100% identical to the DNase sequence into which the alteration was made. Thus, the parent is a DNase having identical amino acid sequence compared to the variant but not having the alterations at one or more of the specified positions. The DNase parent may be a DNase having at least 60%, at least 61%, at least 62%, at least 63%, at least 64%, at least 65%, at least 66%, at least 67%, at least 68%, at least 69%, at least 70%, at least 72%, at least 73%, at least 74%, at least 75%, at least 80%, at least 81%, at least 82%, at least 83%, at least 84%, at least 85%, at least 90%, at least 91%, at least 92%, at least 93%, at least 94%, at least 95%, at least 96%, at least 97%, at least 98%, at least 99% or 100% identity to a polypeptide shown in SEQ ID NO: 1.

The term "relevant washing conditions" is used herein to indicate the conditions, particularly washing temperature, time, washing mechanics, detergent concentration, type of detergent and water hardness, used in households in a detergent market segment.

The term "stability" includes storage stability and stability during use, e.g. during a wash process and reflects the stability of the DNase variant according to the invention as a function of time, e.g. how much activity is retained when the DNase variant is kept in solution in a detergent solution. The stability is influenced by many factors such as pH, temperature, detergent composition, e.g. amount of builder, surfactants etc. The DNase stability may be measured as described in Example 3. The term "improved stability" or "increased stability" is defined herein as a variant DNase displaying an increased stability in solution, e.g. in a detergent solution, relative to the stability of the parent DNase and/or relative to SEQ ID NO: 1. "Improved stability" and "increased stability" include detergent stability during storage or use ("in-wash stability").

The term "textile" refers to woven fabrics, as well as staple fibers and filaments suitable for conversion to or use as yarns, woven, knit, and non-woven fabrics. The term encompasses yarns made from natural as well as synthetic fibers. The term, "textile materials" is a general term for fibers, yarn intermediates, yarn, fabrics, and products made from fabrics (e.g., garments and other articles).

The term "variant" means a polypeptide having DNase activity and which comprises a substitution at one or more positions as defined elsewhere herein. A substitution means replacement of the amino acid occupying a position with a different amino acid, a deletion means removal of an amino acid occupying a position, and an insertion means adding amino acids e.g. 1 to 10 amino acids, preferably 1-3 amino acids, adjacent to an amino acid occupying a position. The variants of the present invention have at least 20%, *e.g.*, at least 40%, at least 50%, at least 60%, at least 70%, at least 80%, at least 90%, at least 95%, or at least 100% of the DNase activity of the polypeptide of SEQ ID NO: 1.

The term "wash liquor" refers to an aqueous solution comprising a DNase variant of the invention. A wash liquor is a solution, e.g. found in a washing machine or dishwasher, containing water and a detergent composition comprising the DNase. The detergent composition, prior to being mixed with water to form a wash liquor, may be in any form as described elsewhere herein, for example a liquid or powder.

The term "water hardness" or "degree of hardness" or "dH" or "°dH" as used herein refers to German degrees of hardness. One degree is defined as 10 milligrams of calcium oxide per liter of water.

### Conventions for Designation of Variants

Unless indicated otherwise, the polypeptide disclosed in SEQ ID NO: 1 is used for purposes of the present invention to determine the corresponding amino acid residue in another DNase. The amino acid sequence of another DNase is aligned with the polypeptide disclosed in SEQ ID NO: 1, and based on the alignment, the amino acid position number corresponding to any amino acid residue in the polypeptide disclosed in SEQ ID NO: 1 is determined using e.g. the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix.

Identification of the corresponding amino acid residue in another DNase can be determined by an alignment of multiple polypeptide sequences using several computer programs including, but not limited to, MUSCLE (multiple sequence comparison by log-expectation; version 3.5 or later; Edgar, 2004, Nucleic Acids Research 32: 1792-1797), MAFFT (version 6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537:_39-64; Katoh and Toh, 2010, Bioinformatics 26: 1899-1900), and EMBOSS EMMA employing ClustalW (1.83 or later; Thompson et al., 1994, Nucleic Acids Research 22: 4673-4680), using their respective default parameters.

When another enzyme has diverged from the polypeptide of SEQ ID NO: 1 such that traditional sequence-based comparison fails to detect their relationship (Lindahl and Elofsson, 2000, J. Mol. Biol. 295: 613-615), other pairwise sequence comparison algorithms can be used. Greater sensitivity in sequence-based searching can be attained using search programs that utilize probabilistic representations of polypeptide families (profiles) to search databases. For example, the PSI-BLAST program generates profiles through an iterative database search process and is capable of detecting remote homologs (Atschul et al., 1997, Nucleic Acids Res. 25: 3389-3402). Even greater sensitivity can be achieved if the family or superfamily for the polypeptide has one or more representatives in the protein structure databases. Programs such as GenTHREADER (Jones, 1999, J. Mol. Biol. 287: 797-815; McGuffin and Jones, 2003, Bioinformatics 19: 874-881) utilize information from a variety of sources (PSI-BLAST, secondary structure prediction, structural alignment profiles, and solvation potentials) as input to a neural network that predicts the structural fold for a query sequence. Similarly, the method of Gough et al., 2000, J. Mol. Biol. 313: 903-919, can be used to align a sequence of unknown structure with the superfamily models present in the SCOP database. These alignments can in turn be used to generate homology models for the polypeptide, and such models can be assessed for accuracy using a variety of tools developed for that purpose.

For proteins of known structure, several tools and resources are available for retrieving and generating structural alignments. For example, the SCOP super families of proteins have been structurally aligned, and those alignments are accessible and downloadable. Two or more protein structures can be aligned using a variety of algorithms such as the distance alignment matrix (Holm and Sander, 1998, Proteins 33: 88-96) or combinatorial extension (Shindyalov and Bourne, 1998, Protein Engineering 11: 739-747), and implementation of these algorithms can additionally be utilized to query structure databases with a structure of interest in order to discover possible structural homologs (*e.g.,* Holm and Park, 2000, Bioinformatics 16: 566-567).

As different amino acids may be present at a given position depending on the selected parent for the variants the amino acid positions are indicated with #₁, #₂, etc. in the definitions below. In describing the variants of the present invention, the nomenclature described below is adapted for ease of reference. The accepted IUPAC single letter or three letters amino acid abbreviations are employed.

Substitutions: For an amino acid substitution, the following nomenclature is used: Original amino acid, position, substituted amino acid. Accordingly, the substitution of valine at position #₁ with alanine is designated as "Val#₁Ala" or "V#₁A". Multiple mutations are separated by addition marks ("+") or by commas (,), *e.g*., "Val#₁Ala + "Pro#₂Gly" or V#₁A, P#₂G, representing substitutions at positions #₁ and #₂ of valine (V) and proline (P) with alanine (A) and glycine (G), respectively. If more than one amino acid may be substituted in a given position these are listed in brackets, such as [X] or {X}. Thus, if both Trp and Lys may be substituted instead of the amino acid occupying at position #₁ this is indicated as X#₁ {W, K}, X#₁ [W, K] or X#₁ [W/K], where the X indicate the amino acid residue present at the position of the parent DNase e.g. such as a DNase shown in SEQ ID NO: 1 or a DNase having at least 60% identity hereto. In some cases, the variants may be represented as #₁ {W, K} or X#₂P indicating that the amino acids to be substituted vary depending on the parent.

For convenience, as SEQ ID NO: 1 is used for numbering the substitutions and other mutations disclosed herein, the amino acid in the corresponding position in SEQ ID NO: 1 is indicated, for example, T65V. However, it will be clear to the skilled artisan that a DNase variant comprising T65V is not limited to parent DNases having threonine at a position corresponding to position 65 of SEQ ID NO: 1. In a parent DNase having e.g. asparagine in position 65, the skilled person would translate the mutation specified herein as T65V to N65V. In the event a parent DNase has valine in position 65, the skilled person would recognize that the parent DNase is not changed at this position. The same applies for deletions and insertions described below. This may also be indicated using an "X", which is defined to be any of the 20 natural amino acids, as the original amino acid, e.g. X65V means that any amino acid residue other than V in position 65 is altered to V.

Deletions: For an amino acid deletion, the following nomenclature is used: Original amino acid, position, *. Accordingly, the deletion of valine at position #₁ is designated as "Val#₁*" or "V#₁*". Multiple deletions are separated by addition marks ("+") or commas, e.g., "Val#₁* + Pro#₂*" or "V#₁*, P#₂*".

Insertions: The insertion of an additional amino acid residue such as e.g. a lysine after Val#₁ may be indicated by: Val#₁ValLys or V#₁VK. Alternatively, insertion of an additional amino acid residue such as lysine after V#₁ may be indicated by: *#₁aK. When more than one amino acid residue is inserted, such as e.g. a Lys, and Gly after #₁ this may be indicated as: Val#₁ValLysGly or V#₁VKG. In such cases, the inserted amino acid residue(s) may also be numbered by the addition of lower case letters to the position number of the amino acid residue preceding the inserted amino acid residue(s), in this example: *#₁aK *#₁bG.

Multiple alterations: Variants comprising multiple alterations are separated by addition marks ("+") or by commas (,), *e.g*., "Val#₁Trp+Pro#₂Gly", "V#₁W, P#₂G" or "V#₁W + P#₂G" representing a substitution of valine and proline at positions #₁ and #₂ with tryptophan and glycine, respectively as described above.

Different alterations: Where different alterations can be introduced at a position, the different alterations may be separated by a comma, *e.g.*, "Val#₁Trp, Lys" or V#₁W, K representing a substitution of valine at position #₁ with tryptophan or lysine. Thus, "Val#₁Trp, Lys + Pro#₂Asp" designates the following variants: "Val#₁Trp+Pro#₂Asp", "Val#₁Lys+Pro#₂Asp" or V#₁W, K + P#₂D.

Different alterations may also be indicated as using the nomenclature [IV] or [I/V], which means that the amino acid at this position may be isoleucine (Ile, I) or valine (Val, V). Likewise, the nomenclature [LVI] and [L/V/I] means that the amino acid at this position may be a leucine (Leu, L), valine (Val, V) or isoleucine (Ile, I), and so forth for other combinations as described herein. For example, T65I/V refers to a substitution of T in position 65 with either I or V.

### Detailed Description of the Invention

The present invention provides novel DNase variants which compared to the polypeptide of SEQ ID NO: 1 comprise at least one substitution selected from the group consisting of N61D, T65I, T65V, S82R, K107Q, T127S, T127V, G149N, S164D and L181S, wherein the variant has at least 60% sequence identity to SEQ ID NO: 1, such as at least 65%, at least 70%, at least 75% or at least 80% sequence identity. In preferred embodiments, the DNase variants of the invention comprise two or more substitutions selected from the group consisting of N61D, T65I, T65V, S82R, K107Q, T127S, T127V, G149N, S164D and L181S.

### DNase variants

In one aspect, the invention provides a DNase variant which compared to the polypeptide of SEQ ID NO: 1 comprises two or more substitutions selected from the group consisting of N61D, T65I, T65V, S82R, K107Q, T127S, T127V, G149N, S164D and L181S, wherein the variant has at least 60% sequence identity to SEQ ID NO: 1, e.g. at least 70%, at least 80%, at least 85%, at least 90% or at least 95% sequence identity, and has DNase activity.

In some embodiments, the DNase variant comprises two, three, four, five or more of said substitutions, typically two, three, four or five, and optionally with one or more additional substitutions described herein.

In one embodiment, the DNase variant of the invention comprises the substitutions N61D+T65I, and optionally one or more additional substitutions described herein.

In one embodiment, the DNase variant of the invention comprises the substitutions N61D+T65V, and optionally one or more additional substitutions described herein.

In one embodiment, the DNase variant of the invention comprises the substitutions N61D+S82R, and optionally one or more additional substitutions described herein.

In one embodiment, the DNase variant of the invention comprises the substitutions N61D+K107Q, and optionally one or more additional substitutions described herein.

In one embodiment, the DNase variant of the invention comprises the substitutions N61D+T127S, and optionally one or more additional substitutions described herein.

In one embodiment, the DNase variant of the invention comprises the substitutions N61D+T127V, and optionally one or more additional substitutions described herein.

In one embodiment, the DNase variant of the invention comprises the substitutions N61D+G149N, and optionally one or more additional substitutions described herein.

In one embodiment, the DNase variant of the invention comprises the substitutions N61D+S164D, and optionally one or more additional substitutions described herein.

In one embodiment, the DNase variant of the invention comprises the substitutions N61D+L181S, and optionally one or more additional substitutions described herein.

In one embodiment, the DNase variant of the invention comprises the substitutions T65I+S82R, and optionally one or more additional substitutions described herein.

In one embodiment, the DNase variant of the invention comprises the substitutions T65I+K107Q, and optionally one or more additional substitutions described herein.

In one embodiment, the DNase variant of the invention comprises the substitutions T65I+T127S, and optionally one or more additional substitutions described herein.

In one embodiment, the DNase variant of the invention comprises the substitutions T65I+T127V, and optionally one or more additional substitutions described herein.

In one embodiment, the DNase variant of the invention comprises the substitutions T65I+G149N, and optionally one or more additional substitutions described herein.

In one embodiment, the DNase variant of the invention comprises the substitutions T65I+S164D, and optionally one or more additional substitutions described herein.

In one embodiment, the DNase variant of the invention comprises the substitutions T65I+L181S, and optionally one or more additional substitutions described herein.

In one embodiment, the DNase variant of the invention comprises the substitutions T65V+S82R, and optionally one or more additional substitutions described herein.

In one embodiment, the DNase variant of the invention comprises the substitutions T65V+K107Q, and optionally one or more additional substitutions described herein.

In one embodiment, the DNase variant of the invention comprises the substitutions T65V+T127S, and optionally one or more additional substitutions described herein.

In one embodiment, the DNase variant of the invention comprises the substitutions T65V+T127V, and optionally one or more additional substitutions described herein.

In one embodiment, the DNase variant of the invention comprises the substitutions T65V+G149N, and optionally one or more additional substitutions described herein.

In one embodiment, the DNase variant of the invention comprises the substitutions T65V+S164D, and optionally one or more additional substitutions described herein.

In one embodiment, the DNase variant of the invention comprises the substitutions T65V+L181S, and optionally one or more additional substitutions described herein.

In one embodiment, the DNase variant of the invention comprises the substitutions S82R+K107Q, and optionally one or more additional substitutions described herein.

In one embodiment, the DNase variant of the invention comprises the substitutions S82R+T127S, and optionally one or more additional substitutions described herein.

In one embodiment, the DNase variant of the invention comprises the substitutions S82R+T127V, and optionally one or more additional substitutions described herein.

In one embodiment, the DNase variant of the invention comprises the substitutions S82R+G149N, and optionally one or more additional substitutions described herein.

In one embodiment, the DNase variant of the invention comprises the substitutions S82R+S164D, and optionally one or more additional substitutions described herein.

In one embodiment, the DNase variant of the invention comprises the substitutions S82R+L181S, and optionally one or more additional substitutions described herein.

In one embodiment, the DNase variant of the invention comprises the substitutions K107Q+T127S, and optionally one or more additional substitutions described herein.

In one embodiment, the DNase variant of the invention comprises the substitutions K107Q+T127V, and optionally one or more additional substitutions described herein.

In one embodiment, the DNase variant of the invention comprises the substitutions K107Q+G149N, and optionally one or more additional substitutions described herein.

In one embodiment, the DNase variant of the invention comprises the substitutions K107Q+S164D, and optionally one or more additional substitutions described herein.

In one embodiment, the DNase variant of the invention comprises the substitutions K107Q+L181S, and optionally one or more additional substitutions described herein.

In one embodiment, the DNase variant of the invention comprises the substitutions T127S+G149N, and optionally one or more additional substitutions described herein.

In one embodiment, the DNase variant of the invention comprises the substitutions T127S+S164D, and optionally one or more additional substitutions described herein.

In one embodiment, the DNase variant of the invention comprises the substitutions T127S+L181S, and optionally one or more additional substitutions described herein.

In one embodiment, the DNase variant of the invention comprises the substitutions T127V+G149N, and optionally one or more additional substitutions described herein.

In one embodiment, the DNase variant of the invention comprises the substitutions T127V+S164D, and optionally one or more additional substitutions described herein.

In one embodiment, the DNase variant of the invention comprises the substitutions T127V+L181S, and optionally one or more additional substitutions described herein.

In one embodiment, the DNase variant of the invention comprises the substitutions G149N+S164D, and optionally one or more additional substitutions described herein.

In one embodiment, the DNase variant of the invention comprises the substitutions G149N+L181S, and optionally one or more additional substitutions described herein.

In one embodiment, the DNase variant of the invention comprises the substitutions S164D+L181S, and optionally one or more additional substitutions described herein.

In a particular embodiment, the DNase variant of the invention comprises the substitutions T65I or T65V together with at least two of the substitutions N61D, S82R, K107Q, T127S, T127V, G149N, S164D and L181S.

In another particular embodiment, the DNase variant of the invention comprises the substitution N61D together with at least two of the substitutions T65I/V, S82R, K107Q, T127S/V, G149N, S164D and L181S, for example two, three or four of said substitutions. In a preferred embodiment, the variant comprises N61D together with at least two of the substitutions T65I/V, S82R, K107Q, T127S and S164D, for example two, three or four of said substitutions.

In another particular embodiment, the DNase variant of the invention comprises the substitution S82R together with at least two of the substitutions N61D, T65I, T65V, K107Q, T127S, T127V, G149N, S164D and L181S, for example two, three or four of said substitutions.

In another particular embodiment, the DNase variant of the invention comprises the substitution K107Q together with at least two of the substitutions N61D, T65I, T65V, S82R, T127S, T127V, G149N, S164D and L181S, for example two, three or four of said substitutions.

In another particular embodiment, the DNase variant of the invention comprises the substitution T127S together with at least two of the substitutions N61D, T65I, T65V, S82R, K107Q, G149N, S164D and L181S, for example two, three or four of said substitutions.

In another particular embodiment, the DNase variant of the invention comprises the substitution G149N together with at least one of the substitutions N61D, T65I, T65V, S82R, K107Q, T127S, T127V, S164D and L181S, for example two, three or four of said substitutions. One such preferred DNase variant comprises the substitutions T65V+G149N.

In another particular embodiment, the DNase variant of the invention comprises the substitution S164D together with at least one of the substitutions N61D, T65I, T65V, S82R, K107Q, T127S, T127V, G149N and L181S, for example two, three or four of said substitutions.

In some embodiments, the variants may further comprise at least one substitution selected from the group consisting of Q14R, Q14W, K21L, P25S, L33K, Q48D, D56I, D56L, S66Y, S68L, Y77T, S102Y, S106A, R109Q, R109T, D116S, D116W, T171W, L181T and L181W, for example one, two or three or more of said substitutions, typically one, two or three.

Thus, the invention also provides a DNase variant which compared to the polypeptide of SEQ ID NO: 1 comprises at least one substitution, e.g. two or more substitutions, selected from the group consisting of N61D, T65I, T65V, S82R, K107Q, T127S, T127V, G149N, S164D and L181S; and at least one substitution, e.g. two or more substitutions, selected from the group consisting of Q14R, Q14W, K21L, P25S, L33K, Q48D, D56I, D56L, S66Y, S68L, Y77T, S102Y, S106A, R109Q, R109T, D116S, D116W, T171W, L181T and L181W; wherein the variant has at least 60% sequence identity to SEQ ID NO: 1, e.g. at least 65%, at least 70%, at least 75%, at least 80%, at least 85%, at least 90% or at least 95% sequence identity, and has DNase activity.

In particular embodiments, the DNase variant comprises a set of substitutions selected from the group consisting of:
- K21L+Q48D+T65I+S82R+K107Q+T127S;
- Q14R+K21L+Q48D+T65I+T127S;
- Q48D+T65I+S82R+T127S+S164D;
- N61D+T65I+K107Q+T127S+S164D;
- Q48D+T65I+S82R+K107Q+T127S;
- Q14R+N61D+T65I+S82R+K107Q;
- N61D+S68L+G149N;
- Q14R+N61D+T65I+S82R+T127S+S164D;
- K21L+Q48D+T65I+S82R+K107Q;
- Q14R+T65I+K107Q+T127S;
- N61D+T65I+S82R+T127S+S164D;
- K21L+N61D+T65I+S82R+K107Q+T127S;
- T65V+T127V+G149N;
- T65I+K107Q+T127S+S164D;
- N61D+T65I+S82R+K107Q;
- Q14R+K21L+N61D+T65I+S82R;
- Q14R+K21L+N61D+T65I+S82R+K107Q;
- Q14R+K21L+N61D+T65I+T127S;
- N61D+T65I+S82R+K107Q+T127S+S164D;
- Q14R+K21L+T65I+K107Q+T127S;
- N61D+T65I+K107Q+T127S;
- T65I+S82R+K107Q+S164D;
- K21L+N61D+T65I+S82R;
- K21L+N61D+T65I+T127S;
- N61D+T65I+S82R+S164D;
- K21L+N61D+T65I+S82R+K107Q;
- S68L+S106A+G149N;
- N61D+T65I+T127S+S164D;
- Q14R+K21L+N61D+T65I;
- Q14R+K21L+T65I+T127S;
- T65V+G149N;
- T65V+R109T+T127V;
- Q14R+K21L+T65I+K107Q;
- K21L+T65I+S82R+K107Q;
- K21L+T65I+K107Q+T127S;
- N61D+S68L+S102Y+G149N+S164D+L181T;
- N61D+S68L+S106A+G149N+S164D;
- T65V+R109T+G149N;
- T65V+T127V+T171W;
- T65V+T127V+L181S;
- T65I+S164D+L181W;
- N61D+S66Y+S102Y+S164D;
- N61D+S66Y+S164D;
- N61D+T65V+S164D;
- Q14W+N61D+T65I;
- Q14W+N61D+T65I+S164D;
- Q14W+N61D+T65I+S164D+L181W;
- T65I+D116W+S164D+L181W;
- T65I+D116W+S164D;
- Q14W+T65I+S164D;
- R109T+G149N;
- G149N+T171W;
- G149N;
- S164D;
- P25S+L33K+D56I+T65V+Y77T+T127V+L181S;
- P25S+L33K+T65V+Y77T+T127V+L181S;
- P25S+L33K+D56I+T65V+Y77T+R109Q+T127V+L181S;
- P25S+L33K+D56I+T65V+Y77T+D116S+T127V+L181S;
- D56L+T65V+T127V;
- D56L+T65V+T127V+T171W;
- T65V+G149N+T171W;
- Q48D+T65I+K107Q+T127S+S164D;
- T65V+Y77T+T127V;
- T65V+R109T+T127V+G149N;
- T65V+R109T+T171W;
- T65V+R109T+G149N+T171W;
- P25S+D56I+T65V+Y77T+T127V+L181S;
- Q14R+K21L+T65I+K107Q+T127S;
- N61D+S68L+S102Y;
- S66Y+T127V+L181S;
- N61D+T65I+S82R+K107Q+L181D;
- T65V+G149N+L181E;
- T65V+T127V+G149N+Y182D;
- Q48D+N61D+T65I+S82R+K107Q;
- Q48D+T65V+G149N;
- N61D+T65I+S82R+T127S+S164D+Y182D;
- N61D+T65I+S82R+T127S+S164D+L181D;
- Q48D+N61D+T65I+K107Q+T127S+S164D;
- N61D+T65I+S82R+T127S+S164D+Y182N;
- T65I+K107Q+T127S+S164D+Y182D;
- N61D+T65I+K107Q+T127S+S164D+L181E;
- N61D+T65I+K107Q+T127S+S164D+L181D;
- T65I+K107Q+T127S+S164D+L181T;
- T65I+K107Q+T127S+S164D+L181E;
- N61D+T65I+K107Q+T127S+S164D+Y182D;
- T65I+K107Q+T127S+S164D+Y182N;
- N61D+T65I+K107Q+T127S+S164D+L181Q;
- N61D+T65V+S164D+Y182N;
- N61D+T65I+K107Q+T127S+S164D+L181T;
- T65I+K107Q+T127S+S164D+L181D;
- T65I+K107Q+T127S+S164D+L181Q;
- N61D+T65V+T127S+S164D;
- Q48D+N61D+T65V+S164D;
- K21L+N61D+T65I+K107Q+T127S;
- Q14W+N61D+T65I+R109T+G149N+S164D+L181W;
- K21L+N61D+T65I+K107Q;
- Q14W+N61D+T65I+R109T+G149N;
- Q14W+T65V+R109T+G149N+W1541+L181W;
- Q14W+T65V+R109T+G149N+W154I+S164D;
- Q14W+N61D+T65V+R109T+G149N+L181W;
- Q14W+T65I+R109T+D116W+G149N+S164D+L181W;
- T65V+R109T+T127V+T171W; and
- R109T+T127V+T171W.

In preferred embodiments, the DNase variant may comprise or consist of SEQ ID NO: 1 with one of the sets of substitutions listed above.

In more preferred embodiments, the DNase variant comprises a set of substitutions selected from the group consisting of:
- T65V+T127V+L181S;
- N61D+T65I+S82R+K107Q;
- T65V+G149N;
- N61D+T65I+K107Q+T127S+S164D;
- N61D+T65I+T127S+S164D;
- N61D+T65V+S164D;
- T65V+T127V+G149N;
- N61D+T65I+S82R+T127S+S164D; and
- T65I+K107Q+T127S+S164D.

In further preferred embodiments, the DNase variant may comprise or consist of SEQ ID NO: 1 with one of the preferred sets of substitutions listed above.

Preferably, the DNase variants of the invention comprise at least 5, such as at least 10, such as at least 15, of the indicated amino acid residues in the following positions: I in position 1, Y in position 13, P in position 22, P in position 25, L in position 27, P in position 39, G in position 42, W in position 57, V in position 59, L in position 76, Y in position 77, R in position 109, D in position 116, P in position 144, H in position 147, L in position 167, D in position 175 and L in position 181.

The DNase variants may e.g. comprise 10, 11, 12, 13, 14, 15, 16, 17 or 18 of the indicated amino acid residues in the following positions: I in position 1, Y in position 13, P in position 22, P in position 25, L in position 27, P in position 39, G in position 42, W in position 57, V in position 59, L in position 76, Y in position 77, R in position 109, D in position 116, P in position 144, H in position 147, L in position 167, D in position 175 and L in position 181.

In other embodiments, a DNase variant of the invention may comprise, in addition to the substitutions disclosed elsewhere herein, one or more substitutions, such as two, three, four or more substitutions, selected from the group consisting of I1T, Y13S, P22T, P25S, L27S, P39S, G42S, W57S, V59S, L76V, Y77T, R109Q, D116S, P144S, H147A, L167S and D175G.

In some embodiments, the DNase variant may comprise additional alterations, typically substitutions, in one or more positions of SEQ ID NO: 1 selected from the group consisting of positions 14, 21, 25, 33, 48, 56, 66, 68, 77, 102, 106, 109, 116, 171 and 181. Preferred substitutions in these positions include one or more substitutions selected from the group consisting of Q14R, Q14W, K21L, P25S, L33K, Q48D, D56I, D56L, S66Y, S68L, Y77T, S102Y, S106A, R109Q, R109T, D116S, D116W, T171W, L181T and L181W.

In one aspect, the invention provides a DNase variant which compared to the polypeptide of SEQ ID NO: 1 comprises a substitution selected from the group consisting of N61D, T65I, T65V, S82R, K107Q, T127S, T127V, G149N, S164D and L181S, and one or more substitutions selected from the group consisting of Q14R, Q14W, K21L, P25S, L33K, Q48D, D56I, D56L, S66Y, S68L, Y77T, S102Y, S106A, R109Q, R109T, D116S, D116W, T171W, L181T and L181W, wherein the variant has at least 60% sequence identity to SEQ ID NO: 1, e.g. at least 70%, at least 80%, at least 85%, at least 90% or at least 95% sequence identity, and has DNase activity.

In one aspect, the invention provides a DNase variant which compared to the polypeptide of SEQ ID NO: 1 comprises the substitution G149N and/or S164D, wherein the variant has at least 60% sequence identity to SEQ ID NO: 1, e.g. at least 70%, at least 80%, at least 85%, at least 90% or at least 95% sequence identity, and has DNase activity.

In one embodiment of this aspect, the DNase variant comprises the substitution G149N, wherein the variant has at least 60% sequence identity to SEQ ID NO: 1, e.g. at least 70%, at least 80%, at least 85%, at least 90% or at least 95% sequence identity, and has DNase activity. The variant may further comprise one or more additional substitutions, e.g. (i) one or more substitutions selected from the group consisting of N61D, T65I, T65V, S82R, K107Q, T127S, T127V, S164D and L181S, and/or (ii) one or more substitutions selected from the group consisting of Q14R, Q14W, K21L, P25S, L33K, Q48D, D56I, D56L, S66Y, S68L, Y77T, S102Y, S106A, R109Q, R109T, D116S, D116W, T171W, L181T and L181W. The DNase variant may further comprises one or more substitutions selected from the group consisting of I1T, Y13S, P22T, P25S, L27S, P39S, G42S, W57S, V59S, L76V, Y77T, R109Q, D116S, P144S, H147A, L167S, D175G and L181S.

In another embodiment of this aspect, the DNase variant comprises the substitution S164D, wherein the variant has at least 60% sequence identity to SEQ ID NO: 1, e.g. at least 70%, at least 80%, at least 85%, at least 90% or at least 95% sequence identity, and has DNase activity. The variant may further comprise one or more additional substitutions, e.g. (i) one or more substitutions selected from the group consisting of N61D, T65I, T65V, S82R, K107Q, T127S, T127V, G149N and L181S, and/or (ii) one or more substitutions selected from the group consisting of Q14R, Q14W, K21L, P25S, L33K, Q48D, D56I, D56L, S66Y, S68L, Y77T, S102Y, S106A, R109Q, R109T, D116S, D116W, T171W, L181T and L181W. The DNase variant may further comprises one or more substitutions selected from the group consisting of I1T, Y13S, P22T, P25S, L27S, P39S, G42S, W57S, V59S, L76V, Y77T, R109Q, D116S, P144S, H147A, L167S, D175G and L181S.

In a further aspect, the invention provides a polypeptide having DNase activity that comprises or consists of the amino acid sequence of SEQ ID NO: 1.

As indicated above, the DNase variants of the invention have at least 60% sequence identity to SEQ ID NO: 1, e.g. at least 70%, at least 75%, at least 80%, at least 85% or at least 90% sequence identity to SEQ ID NO: 1. In further embodiments, any of the DNase variants of the invention may have at least 91%, at least 92%, at least 93%, at least 94%, at least 95%, at least 96%, at least 97%, at least 97%, at least 98% or at least 99% sequence identity to SEQ ID NO: 1.

The DNase variant of the invention may thus comprise more than two alterations, typically substitutions, compared to SEQ ID NO: 1, such as three, four, five, six, seven, eight, nine or ten alterations. In other embodiments, the DNase variant may comprise more than ten alterations, typically substitutions, compared to SEQ ID NO: 1, e.g. up to 15 or up to 20 alterations.

In preferred embodiments, the DNase variants of the invention have an improved property which may be increased stability, e.g. improved detergent stability, improved in-wash stability and/or improved thermostability. Preferably, the DNase variants of the invention have improved stability detergent stability, in particular improved detergent storage stability. In addition, the variants of the invention preferably have substantially maintained or, more preferably, improved relative wash performance compared to a reference DNase, where the reference DNase can e.g. be the DNase of SEQ ID NO: 1 or SEQ ID NO: 27.

The variants according to the invention may comprise additional mutations to the ones listed above. These additional modifications should preferably not significantly change the improved properties of the variant DNase and may e.g. be conservative substitutions.

Examples of conservative substitutions are within the groups of basic amino acids (arginine, lysine and histidine), acidic amino acids (glutamic acid and aspartic acid), polar amino acids (glutamine and asparagine), hydrophobic amino acids (leucine, isoleucine and valine), aromatic amino acids (phenylalanine, tryptophan and tyrosine), and small amino acids (glycine, alanine, serine, threonine and methionine). Amino acid substitutions that do not generally alter specific activity are known in the art and are described, for example, by H. Neurath and R.L. Hill, 1979, In, The Proteins, Academic Press, New York. Common substitutions are Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, and Asp/Gly.

Essential amino acids in a polypeptide can be identified according to procedures known in the art, such as site-directed mutagenesis or alanine-scanning mutagenesis (Cunningham and Wells, 1989, Science 244: 1081-1085). In the latter technique, single alanine mutations are introduced at every residue in the molecule, and the resultant mutant molecules are tested for DNase activity to identify amino acid residues that are critical to the activity of the molecule. See also, Hilton et al., 1996, J. Biol. Chem. 271: 4699-4708. The active site of the enzyme or other biological interaction can also be determined by physical analysis of structure, as determined by such techniques as nuclear magnetic resonance, crystallography, electron diffraction, or photoaffinity labeling, in conjunction with mutation of putative contact site amino acids. See, for example, de Vos et al., 1992, Science 255: 306-312; Smith et al., 1992, J. Mol. Biol. 224: 899-904; Wlodaver et al., 1992, FEBS Lett. 309: 59-64.

### Parent DNase

Preferably the parent DNase is selected from any of the enzyme classes E.C. 3.1.11, E.C. 3.1.12, E.C. 3.1.15, E.C. 3.1.16, E.C. 3.1.21, E.C 3.1.22, E.C 3.1.23, E.C 3.1.24 and E.C.3.1.25.

Preferably, the DNase parent is obtained from a microorganism and the DNase is a microbial enzyme. The DNase is preferably of fungal or bacterial origin.

The DNase parent is preferably obtainable from *Bacillus, such as a Bacillus cibi, Bacillus sp-62451, Bacillus horikoshii, Bacillus sp-16840, Bacillus sp-62668, Bacillus sp-13395, Bacillus horneckiae, Bacillus sp-11238, Bacillus idriensis, Bacillus sp-62520, Bacillus algicola, Bacillus vietnamensis, Bacillus hwajinpoensis, Bacillus indicus, Bacillus marisflavi, Bacillus luciferensis, Bacillus sp. SA2-6.*

The parent DNase preferably belongs to the group of DNases comprised in the GYS-clade, which are DNases comprising the conservative motifs [D/M/L][S/T]GYSR[D/N] (SEQ ID NO: 25) or ASXNRSKG (SEQ ID NO: 26) and which share similar structural and functional properties, see e.g. WO 2017/060475. The DNases of the GYS-clade are preferably obtained from *Bacillus* genus.

One embodiment of the invention relates to a variant of a DNase parent of the GYS-clade having DNase activity, optionally wherein the parent comprises one or both motifs [D/M/L][S/T]GYSR[D/N] (SEQ ID NO: 25), ASXNRSKG (SEQ ID NO: 26), or wherein the parent is a variant of a DNase polypeptide comprising one or both of these motifs, e.g. a variant of a DNase polypeptide obtained from a naturally occurring microorganism, and wherein the polypeptide is selected from the group of polypeptides:
a) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 1,
b) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 2,
c) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 3,
d) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 4,
e) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 5,
f) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 6,
g) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 7,
h) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 8,
i) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 9,
j) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 10,
k) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 11,
l) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 12,
m) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 13,
n) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 14,
o) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 15,
p) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 16,
q) a polypeptide having at 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 17,
r) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 18,
s) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 19,
t) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 20,
u) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 21,
v) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 22,
w) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 23,
x) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 24, and
y) a polypeptide having at least 60%, at least 70%, at least 80%, at least 85%, at least 90%, at least 91%, at least 92%, at least 93%, at least 94%, at least 95%, at least 96%, at least 97%, at least 98%, at least 99% or 100% sequence identity to the polypeptide shown in SEQ ID NO: 27.

Polypeptides having DNase activity and which comprise the GYS-clade motifs have shown particularly good deep cleaning properties, e.g. the DNases are particularly effective in removing or reducing components of organic matter, such as biofilm components, from an item such as a textile or a hard surface.

In a preferred embodiment, the parent has a sequence identity to the polypeptide shown in SEQ ID NO: 1 of at least 60%, at least 65%, at least 70%, at least 75%, at least 80%, at least 85%, at least 90%, at least 95%, such as at least 96%, at least 97%, at least 98%, at least 99% or 100% sequence identity to the polypeptide shown in SEQ ID NO: 1, which has DNase activity.

In some aspects, the parent comprises or consists of the amino acid sequence of SEQ ID NO: 1.

### Use of DNase variants

The DNase variants of the invention are suitable for use in a cleaning process such as laundry or hard surface cleaning. Thus, one aspect of the invention relates a method for laundering an item, wherein the item is a textile, the method comprising:
a) exposing the item to a wash liquor comprising a DNase variant of the invention;
b) completing at least one wash cycle; and optionally
c) rinsing the item.

The pH of the liquid wash liquor solution is typically in the range about 5.5 to about 10, more typically in the range of about 7 to about 9, such as in the range of about 7 to about 8.5 or about 7 to about 8.

The wash liquor may have a temperature in the range of 5°C to 95°C, or in the range of 10°C to 80°C, in the range of 10°C to 70°C, in the range of 10°C to 60°C, in the range of 10°C to 50°C, in the range of 15°C to 40°C or in the range of 20°C to 30°C.

The concentration of the DNase variant enzyme in the wash liquor is typically in the range of from 0.00001 ppm to 10 ppm enzyme protein, from 0.00002 ppm to 10 ppm, from 0.0001 ppm to 10 ppm, from 0.0002 ppm to 10 ppm, from 0.001 ppm to 10 ppm, from 0.002 ppm to 10 ppm, from 0.01 ppm to 10 ppm, from 0.02 ppm to 10 ppm, from 0.1 ppm to 10 ppm, from 0.2 ppm to 10 ppm, or from 0.5 ppm to 5 ppm.

In some aspects, the DNase variants of the invention are effective in preventing and/or reducing malodor it items such as textiles. The presence of biofilm results in laundry items becoming sticky, leading soil to adhere to the sticky areas. This soil stuck to the laundry has been shown to be difficult to remove by commercially available detergent compositions. Further, when dirty laundry items are washed together with less dirty laundry items, the dirt present in the wash liquor tends to adhere to the laundry (referred to as re-deposition) if the laundry is sticky as described above. Thus, laundry items may become more "soiled" after wash than before wash. In some aspects, the DNase variants of the invention have improved deep cleaning properties compared to the parent DNase, e.g. reducing stickiness and/or re-deposition.

The DNase variants according to the invention may thus be used for deep cleaning of an item, wherein the item is a fabric or a hard surface.

The invention thus also relates to use of a DNase variant according to the invention for preventing and/or reducing adherence of soil to an item, where the item is e.g. a textile. By reducing adherence of soil to an item, the item appears cleaner, and may be said to have improved "whiteness". Thus, the invention further relates to the use of a DNase variant according to the invention for maintaining or improving the whiteness of an item, e.g. a textile.

The present invention further relates to detergent compositions comprising a DNase variant according to the invention together with at least one detergent adjunct ingredient. The detergent composition comprising a DNase variant according to the invention may be used for cleaning, e.g. deep cleaning, of an item, for preventing and/or reducing stickiness of an item, for pretreating stains on an item, for preventing and/or reducing redeposition of soil during a wash cycle, for preventing and/or reducing adherence of soil to an item, for maintaining or improving whiteness of an item, and/or for preventing and/or reducing malodor of an item.

### Preparation of Variants

The present invention also relates to a method for obtaining a DNase variant having at least one improved property compared to the parent DNase e.g. compared to the polypeptide shown in SEQ ID NO: 1.

This aspect thus relates to a method for obtaining a DNase variant, comprising:
a) introducing into a parent DNase having at least 60% sequence identity to SEQ ID NO: 1 two or more substitutions selected from the group consisting of N61D, T65I, T65V, S82R, K107Q, T127S, T127V, G149N, S164D and L181S (wherein numbering is based on SEQ ID NO: 1), and
b) recovering the variant
wherein the variant has DNase activity.

It will be understood that the method of obtaining a DNase variant may further include introduction of any of the other substitutions or combinations of substitutions described above. For example, the method for obtaining a DNase variant may further comprise introduction of at least one substitution selected from the group consisting of Q14R, Q14W, K21L, P25S, L33K, Q48D, D56I, D56L, S66Y, S68L, Y77T, S102Y, S106A, R109Q, R109T, D116S, D116W, T171W, L181T and L181W. It may similarly comprise introduction of at least one substitution selected from the group consisting of I1T, Y13S, P22T, P25S, L27S, P39S, G42S, W57S, V59S, L76V, Y77T, R109Q, D116S, P144S, H147A, L167S and D175G.

In a preferred embodiment, the parent DNase has at least 80% sequence identity to the polypeptide of SEQ ID NO: 1, such as at least 85%, at least 90%, at least 95%, at least 96%, at least 97%, at least 98%, or at least 99% sequence identity to the polypeptide of SEQ ID NO: 1. In one embodiment, the parent DNase has the amino acid sequence of SEQ ID NO: 1.

In other embodiments, the variant may be based on a parent DNase selected from those set forth above, i.e. a polypeptide selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26 or SEQ ID NO: 27; or a polypeptide having at least 60%, at least 70%, at least 80%, at least 85%, at least 90%, at least 91%, at least 92%, at least 93%, at least 94%, at least 95%, at least 96%, at least 97%, at least 98% or at least 99% sequence identity to any of these.

The variants of the invention may be prepared by procedures that are well-known in the art such as those mentioned below.

Site-directed mutagenesis is a technique in which mutations are introduced at one or more defined sites in a polynucleotide encoding the parent. Site-directed mutagenesis can be accomplished *in vitro* by PCR involving the use of oligonucleotide primers containing the desired mutation. Site-directed mutagenesis can also be performed *in vitro* by cassette mutagenesis involving the cleavage by a restriction enzyme at a site in the plasmid comprising a polynucleotide encoding the parent and subsequent ligation of an oligonucleotide containing the mutation in the polynucleotide. Usually the restriction enzyme that digests the plasmid and the oligonucleotide is the same, permitting sticky ends of the plasmid and the insert to ligate to one another. See, *e.g.*, Scherer and Davis, 1979, Proc. Natl. Acad. Sci. USA 76: 4949-4955; and Barton et al., 1990, Nucleic Acids Res. 18: 7349-4966.

Site-directed mutagenesis can also be accomplished *in vivo* by methods known in the art. See, *e.g.,* U.S. Patent Application Publication No. 2004/0171154; Storici et al., 2001, Nature Biotechnol. 19: 773-776; Kren et al., 1998, Nat. Med. 4: 285-290; and Calissano and Macino, 1996, Fungal Genet. Newslett. 43: 15-16.

Synthetic gene construction entails *in vitro* synthesis of a designed polynucleotide molecule to encode a polypeptide of interest. Gene synthesis can be performed utilizing several techniques, such as the multiplex microchip-based technology described by Tian et al. (2004, Nature 432: 1050-1054) and similar technologies wherein oligonucleotides are synthesized and assembled upon photo-programmable microfluidic chips.

Single or multiple amino acid substitutions, deletions, and/or insertions can be made and tested using known methods of mutagenesis, recombination and/or shuffling, followed by a relevant screening procedure, such as those disclosed by Reidhaar-Olson and Sauer, 1988, Science 241: 53-57; Bowie and Sauer, 1989, Proc. Natl. Acad. Sci. USA 86: 2152-2156; WO 95/17413; or WO 95/22625. Other methods that can be used include error-prone PCR, phage display (*e.g.,* Lowman et al., 1991, Biochemistry 30: 10832-10837; US 5,223,409; WO 92/06204) and region-directed mutagenesis (Derbyshire et al., 1986, Gene 46: 145; Ner et a/., 1988, DNA 7: 127).

Mutagenesis/shuffling methods can be combined with high-throughput, automated screening methods to detect activity of cloned, mutagenized polypeptides expressed by host cells (Ness et al., 1999, Nature Biotechnology 17: 893-896). Mutagenized DNA molecules that encode active polypeptides can be recovered from the host cells and rapidly sequenced using standard methods in the art. These methods allow the rapid determination of the importance of individual amino acid residues in a polypeptide.

Semi-synthetic gene construction is accomplished by combining aspects of synthetic gene construction, and/or site-directed mutagenesis, and/or random mutagenesis, and/or shuffling. Semi-synthetic construction is typified by a process utilizing polynucleotide fragments that are synthesized, in combination with PCR techniques. Defined regions of genes may thus be synthesized *de novo,* while other regions may be amplified using site-specific mutagenic primers, while yet other regions may be subjected to error-prone PCR or non-error prone PCR amplification. Polynucleotide subsequences may then be shuffled.

### Nucleic Acid Constructs

The present invention also relates to nucleic acid constructs comprising a polynucleotide encoding the DNase variants of the present invention operably linked to one or more control sequences that direct the expression of the coding sequence in a suitable host cell under conditions compatible with the control sequences.

The polynucleotide may be manipulated in a variety of ways to provide for expression of the polypeptide. Manipulation of the polynucleotide prior to its insertion into a vector may be desirable or necessary depending on the expression vector. The techniques for modifying polynucleotides utilizing recombinant DNA methods are well known in the art.

The control sequence may be a promoter, a polynucleotide that is recognized by a host cell for expression of a polynucleotide encoding a polypeptide of the present invention. The promoter contains transcriptional control sequences that mediate the expression of the polypeptide. The promoter may be any polynucleotide that shows transcriptional activity in the host cell including mutant, truncated, and hybrid promoters, and may be obtained from genes encoding extracellular or intracellular polypeptides either homologous or heterologous to the host cell.

Examples of suitable promoters for directing transcription of the nucleic acid constructs of the present invention in a bacterial host cell are the promoters obtained from the Bacillus amyloliquefaciens alpha-amylase gene (amyQ), Bacillus licheniformis alpha-amylase gene (amyL), Bacillus licheniformis penicillinase gene (penP), Bacillus stearothermophilus maltogenic amylase gene (amyM), Bacillus subtilis levansucrase gene (sacB), Bacillus subtilis xylA and xylB genes, Bacillus thuringiensis cryIIIA gene (Agaisse and Lereclus, 1994, Molecular Microbiology 13: 97-107), E. coli lac operon, E. coli trc promoter (Egon et al., 1988, Gene 69: 301-315), Streptomyces coelicolor agarase gene (dagA), and prokaryotic beta-lactamase gene (Villa-Kamaroff et al., 1978, Proc. Natl. Acad. Sci. USA 75: 3727-3731), as well as the tac promoter (DeBoer et al., 1983, Proc. Natl. Acad. Sci. USA 80: 21-25). Further promoters are described in "Useful proteins from recombinant bacteria" in Gilbert et al., 1980, Scientific American 242: 74-94; and in Sambrook et al., 1989, supra. Examples of tandem promoters are disclosed in WO 99/43835.

Examples of suitable promoters for directing transcription of the nucleic acid constructs of the present invention in a filamentous fungal host cell are promoters obtained from the genes for *Bacillus nidulans* acetamidase, *Bacillus niger* neutral alpha-amylase, *Bacillus niger* acid stable alpha-amylase, *Bacillus niger* or *Bacillus awamori* glucoamylase (glaA), *Bacillus cibi* TAKA amylase, *Bacillus cibi* alkaline protease, *Bacillus cibi* triose phosphate isomerase, *Fusarium oxysporum* trypsin-like protease (WO 96/00787), *Fusarium venenatum* amyloglucosidase (WO 00/56900), *Fusarium venenatum* Daria (WO 00/56900), *Fusarium venenatum* Quinn (WO 00/56900), *Rhizomucor miehei* lipase, *Rhizomucor miehei* aspartic proteinase, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor, as well as the NA2 tpi promoter (a modified promoter from a *Bacillus* neutral alpha-amylase gene in which the untranslated leader has been replaced by an untranslated leader from a *Bacillus triose* phosphate isomerase gene; non-limiting examples include modified promoters from a *Bacillus niger* neutral alpha-amylase gene in which the untranslated leader has been replaced by an untranslated leader from a *Bacillus nidulans* or *Bacillus cibi* triose phosphate isomerase gene); and mutant, truncated, and hybrid promoters thereof. Other promoters are described in U.S. Patent No. 6,011,147.

In a yeast host, useful promoters are obtained from the genes for *Saccharomyces cerevisiae* enolase (ENO 1), *Saccharomyces cerevisiae* galactokinase (GAL1), *Saccharomyces cerevisiae* alcohol dehydrogenase/glyceraldehyde-3 phosphate dehydrogenase (ADH1, ADH2/GAP), *Saccharomyces cerevisiae* triose phosphate isomerase (TPI), *Saccharomyces cerevisiae* metallothionein (CUP1), and *Saccharomyces cerevisiae* 3 phosphoglycerate kinase. Other useful promoters for yeast host cells are described by Romanos et al., 1992, Yeast 8: 423-488.

The control sequence may also be a transcription terminator, which is recognized by a host cell to terminate transcription. The terminator is operably linked to the 3' terminus of the polynucleotide encoding the polypeptide. Any terminator that is functional in the host cell may be used in the present invention.

Preferred terminators for bacterial host cells are obtained from the genes for *Bacillus clausii* alkaline protease (aprH), *Bacillus licheniformis* alpha-amylase (amyL), and *Escherichia coli* ribosomal RNA (rrnB). Other terminators may be obtained from the genes for *Bacillus nidulans* acetamidase, *Bacillus nidulans* anthranilate synthase, *Bacillus niger* glucoamylase, *Bacillus niger* alpha-glucosidase or *Bacillus cibi* TAKA amylase.

Preferred terminators for filamentous fungal host cells are obtained from the genes for *Aspergillus nidulans* acetamidase, *Aspergillus nidulans* anthranilate synthase, *Aspergillus niger* glucoamylase, *Aspergillus niger* alpha-glucosidase, *Aspergillus oryzae* TAKA amylase, *Fusarium oxysporum* trypsin-like protease, *Trichoderma reesei* beta-glucosidase, *Trichoderma reesei* cellobiohydrolase I, *Trichoderma reesei* cellobiohydrolase II, *Trichoderma reesei* endoglucanase I, *Trichoderma reesei* endoglucanase II, *Trichoderma reesei* endoglucanase III, *Trichoderma reesei* endoglucanase V, *Trichoderma reesei* xylanase I, *Trichoderma reesei* xylanase II, *Trichoderma reesei* xylanase III, *Trichoderma reesei* beta-xylosidase, and *Trichoderma reesei* translation elongation factor.

Preferred terminators for yeast host cells are obtained from the genes for *Saccharomyces cerevisiae* enolase, *Saccharomyces cerevisiae* cytochrome C (CYC1), and *Saccharomyces cerevisiae* glyceraldehyde-3 phosphate dehydrogenase. Other useful terminators for yeast host cells are described by Romanos et al., 1992, supra.

The control sequence may also be an mRNA stabilizer region downstream of a promoter and upstream of the coding sequence of a gene which increases expression of the gene. Examples of suitable mRNA stabilizer regions are obtained from a *Bacillus thuringiensis* cryIIIA gene (WO 94/25612) and a Bacillus subtilis SP82 gene (Hue et al., 1995, Journal of Bacteriology 177: 3465-3471).

The control sequence may also be a leader, a nontranslated region of an mRNA that is important for translation by the host cell. The leader is operably linked to the 5' terminus of the polynucleotide encoding the polypeptide. Any leader that is functional in the host cell may be used.

Preferred leaders for filamentous fungal host cells are obtained from the genes for *Bacillus cibi* TAKA amylase and *Bacillus nidulans* triose phosphate isomerase.

Suitable leaders for yeast host cells are obtained from the genes for *Saccharomyces cerevisiae* enolase (ENO 1), *Saccharomyces cerevisiae* 3 phosphoglycerate kinase, *Saccharomyces cerevisiae* alpha-factor, and *Saccharomyces cerevisiae* alcohol dehydrogenase/glyceraldehyde-3 phosphate dehydrogenase (ADH2/GAP).

The control sequence may also be a polyadenylation sequence, a sequence operably linked to the 3' terminus of the polynucleotide and, when transcribed, is recognized by the host cell as a signal to add polyadenosine residues to transcribed mRNA. Any polyadenylation sequence that is functional in the host cell may be used.

Preferred polyadenylation sequences for filamentous fungal host cells are obtained from the genes for *Bacillus nidulans* anthranilate synthase, *Bacillus* nigerglucoamylase, *Bacillus niger* alpha-glucosidase Bacillus cibi TAKA amylase, and *Fusarium oxysporum* trypsin-like protease.

Useful polyadenylation sequences for yeast host cells are described by Guo and Sherman, 1995, Mol. Cellular Biol. 15: 5983-5990.

The control sequence may also be a signal peptide coding region that encodes a signal peptide linked to the N terminus of a polypeptide and directs the polypeptide into the cell's secretory pathway. The 5' end of the coding sequence of the polynucleotide may inherently contain a signal peptide coding sequence naturally linked in translation reading frame with the segment of the coding sequence that encodes the polypeptide. Alternatively, the 5' end of the coding sequence may contain a signal peptide coding sequence that is foreign to the coding sequence. A foreign signal peptide coding sequence may be required where the coding sequence does not naturally contain a signal peptide coding sequence. Alternatively, a foreign signal peptide coding sequence may simply replace the natural signal peptide coding sequence in order to enhance secretion of the polypeptide. However, any signal peptide coding sequence that directs the expressed polypeptide into the secretory pathway of a host cell may be used.

Effective signal peptide coding sequences for bacterial host cells are the signal peptide coding sequences obtained from the genes for *Bacillus* NCIB 11837 maltogenic amylase, *Bacillus licheniformis* subtilisin, *Bacillus licheniformis* beta-lactamase, *Bacillus stearothermophilus* alpha-amylase, *Bacillus stearothermophilus* neutral proteases (nprT, nprS, nprM), and *Bacillus subtilis* prsA. Further signal peptides are described by Simonen and Palva, 1993, Microbiological Reviews 57: 109-137.

Effective signal peptide coding sequences for filamentous fungal host cells are the signal peptide coding sequences obtained from the genes for *Aspergillus niger* neutral amylase, *Aspergillus* nigerglucoamylase, *Aspergillus oryzae* TAKA amylase, *Humicola insolens* cellulase, *Humicola insolens* endoglucanase V, *Humicola lanuginosa* lipase, and *Rhizomucor miehei* aspartic proteinase.

Useful signal peptides for yeast host cells are obtained from the genes for *Saccharomyces cerevisiae* alpha-factor and *Saccharomyces cerevisiae* invertase. Other useful signal peptide coding sequences are described by Romanos *et al.,* 1992, *supra.*

The control sequence may also be a propeptide coding sequence that encodes a propeptide positioned at the N-terminus of a polypeptide. The resultant polypeptide is known as a proenzyme or propolypeptide (or a zymogen in some cases). A propolypeptide is generally inactive and can be converted to an active polypeptide by catalytic or autocatalytic cleavage of the propeptide from the propolypeptide. The propeptide coding sequence may be obtained from the genes for *Bacillus subtilis* alkaline protease (*aprE*)*, Bacillus subtilis* neutral protease (*nprT*)*, Myceliophthora thermophila* laccase (WO 95/33836), *Rhizomucor miehei* aspartic proteinase, and *Saccharomyces cerevisiae* alpha-factor.

Where both signal peptide and propeptide sequences are present, the propeptide sequence is positioned next to the N terminus of a polypeptide and the signal peptide sequence is positioned next to the N terminus of the propeptide sequence.

It may also be desirable to add regulatory sequences that regulate expression of the polypeptide relative to the growth of the host cell. Examples of regulatory sequences are those that cause expression of the gene to be turned on or off in response to a chemical or physical stimulus, including the presence of a regulatory compound. Regulatory sequences in prokaryotic systems include the *lac, tac,* and *trp* operator systems. In yeast, the ADH2 system or GAL1 system may be used. In filamentous fungi, the *Aspergillus niger* glucoamylase promoter, *Aspergillus oryzae* TAKA alpha-amylase promoter, and *Aspergillus oryzae* glucoamylase promoter, *Trichoderma reesei* cellobiohydrolase I promoter, and *Trichoderma reesei* cellobiohydrolase II promoter may be used. Other examples of regulatory sequences are those that allow for gene amplification. In eukaryotic systems, these regulatory sequences include the dihydrofolate reductase gene that is amplified in the presence of methotrexate, and the metallothionein genes that are amplified with heavy metals. In these cases, the polynucleotide encoding the polypeptide would be operably linked to the regulatory sequence.

### Expression Vectors

The present invention also relates to recombinant expression vectors comprising a polynucleotide encoding the DNase variants of the present invention, a promoter, and transcriptional and translational stop signals. The various nucleotide and control sequences may be joined together to produce a recombinant expression vector that may include one or more convenient restriction sites to allow for insertion or substitution of the polynucleotide encoding the polypeptide at such sites. Alternatively, the polynucleotide may be expressed by inserting the polynucleotide or a nucleic acid construct comprising the polynucleotide into an appropriate vector for expression. In creating the expression vector, the coding sequence is located in the vector so that the coding sequence is operably linked with the appropriate control sequences for expression. In a further aspect, polynucleotide sequence codons have been modified by nucleotide substitutions to correspond to the codon usage of the host organism intended for production of the polypeptide of the present invention. The recombinant expression vector may be any vector (e.g., a plasmid or virus) that can be conveniently subjected to recombinant DNA procedures and can bring about expression of the polynucleotide. The choice of the vector will typically depend on the compatibility of the vector with the host cell into which the vector is to be introduced. The vector may be a linear or closed circular plasmid.

The vector may be an autonomously replicating vector, i.e., a vector that exists as an extrachromosomal entity, the replication of which is independent of chromosomal replication, e.g., a plasmid, an extrachromosomal element, a minichromosome, or an artificial chromosome. The vector may contain any means for assuring self-replication. Alternatively, the vector may be one that, when introduced into the host cell, is integrated into the genome and replicated together with the chromosome(s) into which it has been integrated. Furthermore, a single vector or plasmid or two or more vectors or plasmids that together contain the total DNA to be introduced into the genome of the host cell, or a transposon, may be used.

The vector preferably contains one or more selectable markers that permit easy selection of transformed, transfected, transduced, or the like cells. A selectable marker is a gene the product of which provides for biocide or viral resistance, resistance to heavy metals, prototrophy to auxotrophs, and the like.

Examples of bacterial selectable markers are *Bacillus licheniformis* or *Bacillus subtilis dal* genes, or markers that confer antibiotic resistance such as ampicillin, chloramphenicol, kanamycin, neomycin, spectinomycin, or tetracycline resistance. Suitable markers for yeast host cells include, but are not limited to, ADE2, HIS3, LEU2, LYS2, MET3, TRP1, and URA3. Selectable markers for use in a filamentous fungal host cell include, but are not limited to, *adeA* (phosphoribosylaminoimidazole-succinocarboxamide synthase), *adeB* (phosphoribosylaminoimidazole synthase), *amdS* (acetamidase), *argB* (ornithine carbamoyltransferase), *bar* (phosphinothricin acetyltransferase), *hph* (hygromycin phosphotransferase), *niaD* (nitrate reductase), *pyrG* (orotidine-5'-phosphate decarboxylase), sC (sulfate adenyltransferase), and *trpC* (anthranilate synthase), as well as equivalents thereof. Preferred for use in an *Aspergillus* cell are *Aspergillus nidulans* or *Aspergillus oryzae amdS* and *pyrG* genes and a *Streptomyces hygroscopicus bar* gene. Preferred for use in a *Trichoderma* cell are *adeA, adeB, amdS, hph,* and *pyrG* genes.

The selectable marker may be a dual selectable marker system as described in WO 2010/039889. In one aspect, the dual selectable marker is a *hph-tk* dual selectable marker system.

The vector preferably contains an element(s) that permits integration of the vector into the host cell's genome or autonomous replication of the vector in the cell independent of the genome. For integration into the host cell genome, the vector may rely on the polynucleotide's sequence encoding the polypeptide or any other element of the vector for integration into the genome by homologous or non-homologous recombination. Alternatively, the vector may contain additional polynucleotides for directing integration by homologous recombination into the genome of the host cell at a precise location(s) in the chromosome(s). To increase the likelihood of integration at a precise location, the integrational elements should contain a sufficient number of nucleic acids, such as 100 to 10,000 base pairs, 400 to 10,000 base pairs, and 800 to 10,000 base pairs, which have a high degree of sequence identity to the corresponding target sequence to enhance the probability of homologous recombination. The integrational elements may be any sequence that is homologous with the target sequence in the genome of the host cell. Furthermore, the integrational elements may be non-encoding or encoding polynucleotides. On the other hand, the vector may be integrated into the genome of the host cell by non-homologous recombination.

For autonomous replication, the vector may further comprise an origin of replication enabling the vector to replicate autonomously in the host cell in question. The origin of replication may be any plasmid replicator mediating autonomous replication that functions in a cell. The term "origin of replication" or "plasmid replicator" means a polynucleotide that enables a plasmid or vector to replicate in vivo.

Examples of bacterial origins of replication are the origins of replication of plasmids pBR322, pUC19, pACYC177, and pACYC184 permitting replication in E. coli, and pUB110, pE194, pTA1060, and pAMβ1 permitting replication in Bacillus.
Examples of origins of replication for use in a yeast host cell are the 2 micron origin of replication, ARS1, ARS4, the combination of ARS1 and CEN3, and the combination of ARS4 and CEN6.

Examples of origins of replication useful in a filamentous fungal cell are AMA1 and ANS1 (Gems et al., 1991, Gene 98: 61-67; Cullen et al., 1987, Nucleic Acids Res. 15: 9163-9175; WO 00/24883). Isolation of the AMA1 gene and construction of plasmids or vectors comprising the gene can be accomplished according to the methods disclosed in WO 00/24883.

More than one copy of a polynucleotide of the present invention may be inserted into a host cell to increase production of a polypeptide. An increase in the copy number of the polynucleotide can be obtained by integrating at least one additional copy of the sequence into the host cell genome or by including an amplifiable selectable marker gene with the polynucleotide where cells containing amplified copies of the selectable marker gene, and thereby additional copies of the polynucleotide, can be selected for by cultivating the cells in the presence of the appropriate selectable agent.

The procedures used to ligate the elements described above to construct the recombinant expression vectors of the present invention are well known to one skilled in the art (see, e.g., Sambrook et al., 1989, supra).

### Host Cells

The present invention also relates to recombinant host cells, comprising a polynucleotide of the present invention operably linked to one or more control sequences that direct the production of a polypeptide of the present invention. A construct or vector comprising a polynucleotide is introduced into a host cell so that the construct or vector is maintained as a chromosomal integrant or as a self-replicating extra-chromosomal vector as described earlier. The term "host cell" encompasses any progeny of a parent cell that is not identical to the parent cell due to mutations that occur during replication. The choice of a host cell will to a large extent depend upon the gene encoding the polypeptide and its source.

The host cell may be any cell useful in the recombinant production of a polypeptide of the present invention, e.g., a prokaryote or a eukaryote.

The prokaryotic host cell may be any Gram-positive or Gram-negative bacterium. Gram-positive bacteria include, but are not limited to, *Bacillus, Clostridium, Enterococcus, Geobacillus, Lactobacillus, Lactococcus, Oceanobacillus, Staphylococcus, Streptococcus,* and *Streptomyces.* Gram-negative bacteria include, but are not limited to, *Campylobacter, E. coli, Flavobacterium, Fusobacterium, Helicobacter, Ilyobacter, Neisseria, Pseudomonas, Salmonella,* and *Ureaplasma.*

The bacterial host cell may be any *Bacillus* cell including, but not limited to, *Bacillus alkalophilus, Bacillus amyloliquefaciens, Bacillus brevis, Bacillus circulans, Bacillus clausii, Bacillus coagulans, Bacillus firmus, Bacillus lautus, Bacillus lentus, Bacillus licheniformis, Bacillus megaterium, Bacillus pumilus, Bacillus stearothermophilus, Bacillus subtilis,* and *Bacillus thuringiensis* cells.

The bacterial host cell may also be any *Streptococcus* cell including, but not limited to, *Streptococcus equisimilis, Streptococcus pyogenes, Streptococcus uberis,* and *Streptococcus equi subsp. Zooepidemicus* cells.

The bacterial host cell may also be any *Streptomyces* cell including, but not limited to, *Streptomyces achromogenes, Streptomyces avermitilis, Streptomyces coelicolor, Streptomyces griseus,* and *Streptomyces lividans* cells.

The introduction of DNA into a Bacillus cell may be effected by protoplast transformation (see, e.g., Chang and Cohen, 1979, Mol. Gen. Genet. 168: 111-115), competent cell transformation (see, e.g., Young and Spizizen, 1961, J. Bacteriol. 81: 823-829, or Dubnau and Davidoff-Abelson, 1971, J. Mol. Biol. 56: 209-221), electroporation (see, e.g., Shigekawa and Dower, 1988, Biotechniques 6: 742-751), or conjugation (see, e.g., Koehler and Thorne, 1987, J. Bacteriol. 169: 5271-5278). The introduction of DNA into an E. coli cell may be effected by protoplast transformation (see, e.g., Hanahan, 1983, J. Mol. Biol. 166: 557-580) or electroporation (see, e.g., Dower et al., 1988, Nucleic Acids Res. 16: 6127-6145). The introduction of DNA into a Streptomyces cell may be effected by protoplast transformation, electroporation (see, e.g., Gong et al., 2004, Folia Microbiol. (Praha) 49: 399-405), conjugation (see, e.g., Mazodier et al., 1989, J. Bacteriol. 171: 3583-3585), or transduction (see, e.g., Burke et al., 2001, Proc. Natl. Acad. Sci. USA 98: 6289-6294). The introduction of DNA into a Pseudomonas cell may be effected by electroporation (see, e.g., Choi et al., 2006, J. Microbiol. Methods 64: 391-397) or conjugation (see, e.g., Pinedo and Smets, 2005, Appl. Environ. Microbiol. 71: 51-57). The introduction of DNA into a Streptococcus cell may be effected by natural competence (see, e.g., Perry and Kuramitsu, 1981, Infect. Immun. 32: 1295-1297), protoplast transformation (see, e.g., Catt and Jollick, 1991, Microbios 68: 189-207), electroporation (see, e.g., Buckley et al., 1999, Appl. Environ. Microbiol. 65: 3800-3804), or conjugation (see, e.g., Clewell, 1981, Microbiol. Rev. 45: 409-436). However, any method known in the art for introducing DNA into a host cell can be used.

The host cell may also be a eukaryote, such as a mammalian, insect, plant, or fungal cell.

The host cell may be a fungal cell. "Fungi" as used herein cludes the phyla Ascomycota, Basidiomycota, Chytridiomycota, and Zygomycota as well as the Oomycota and all mitosporic fungi (as defined by Hawksworth et al., In, Ainsworth and Bisby's Dictionary of The Fungi, 8th edition, 1995, CAB International, University Press, Cambridge, UK).

The fungal host cell may be a yeast cell. "Yeast" as used herein includes ascosporogenous yeast (*Endomycetales*)*,* basidiosporogenous yeast, and yeast belonging to the Fungi Imperfecti (*Blastomycetes*)*.* Since the classification of yeast may change in the future, for the purposes of this invention, yeast shall be defined as described in Biology and Activities of Yeast (Skinner, Passmore, and Davenport, editors, Soc. App. Bacteriol. Symposium Series No. 9, 1980).

The yeast host cell may be a *Candida, Hansenula, Kluyveromyces, Pichia, Saccharomyces, Schizosaccharomyces,* or *Yarrowia* cell, such as a *Kluyveromyces lactis, Saccharomyces carlsbergensis, Saccharomyces cerevisiae, Saccharomyces diastaticus, Saccharomyces douglasii, Saccharomyces kluyveri, Saccharomyces norbensis, Saccharomyces oviformis,* or *Yarrowia lipolytica* cell.

The fungal host cell may be a filamentous fungal cell. "Filamentous fungi" include all filamentous forms of the subdivision Eumycota and Oomycota (as defined by Hawksworth et al., 1995, supra). The filamentous fungi are generally characterized by a mycelial wall composed of chitin, cellulose, glucan, chitosan, mannan, and other complex polysaccharides. Vegetative growth is by hyphal elongation and carbon catabolism is obligately aerobic. In contrast, vegetative growth by yeasts such as *Saccharomyces cerevisiae* is by budding of a unicellular thallus and carbon catabolism may be fermentative.

The filamentous fungal host cell may be an *Acremonium, Bacillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes,* or *Trichoderma* cell.

For example, the filamentous fungal host cell may be an *Aspergillus awamori, Aspergillus foetidus, Aspergillus fumigatus, Aspergillus japonicus, Aspergillus nidulans, Aspergillus niger, Aspergillus oryzae, Bjerkandera adusta, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora, Chrysosporium inops, Chrysosporium keratinophilum, Chrysosporium lucknowense, Chrysosporium merdarium, Chrysosporium pannicola, Chrysosporium queenslandicum, Chrysosporium tropicum, Chrysosporium zonatum, Coprinus cinereus, Coriolus hirsutus, Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides, Fusarium venenatum, Humicola insolens, Humicola lanuginosa, Mucor miehei, Myceliophthora thermophila, Neurospora crassa, Penicillium purpurogenum, Phanerochaete chrysosporium, Phlebia radiata, Pleurotus eryngii, Talaromyces emersonii, Thielavia terrestris, Trametes villosa, Trametes versicolor, Trichoderma harzianum, Trichoderma koningii, Trichoderma longibrachiatum, Trichoderma reesei,* or *Trichoderma viride* cell.

Fungal cells may be transformed by a process involving protoplast formation, transformation of the protoplasts, and regeneration of the cell wall in a manner known *per se.* Suitable procedures for transformation of *Aspergillus* and *Trichoderma* host cells are described in EP 238023, Yelton et al., 1984, Proc. Natl. Acad. Sci. USA 81: 1470-1474, and Christensen et al., 1988, Bio/Technology 6: 1419-1422. Suitable methods for transforming *Fusarium* species are described by Malardier et al., 1989, Gene 78: 147-156, and WO 96/00787. Yeast may be transformed using the procedures described by Becker and Guarente, In Abelson, J.N. and Simon, M.I., editors, Guide to Yeast Genetics and Molecular Biology, Methods in Enzymology, Volume 194, pp 182-187, Academic Press, Inc., New York; Ito et al., 1983, J. Bacteriol. 153: 163; and Hinnen et al., 1978, Proc. Natl. Acad. Sci. USA 75: 1920.

### Methods of Production

The present invention also relates to methods of producing a DNase variant polypeptide of the invention, comprising (a) cultivating a recombinant host cell as described above under conditions conducive for production of the polypeptide; and optionally, (b) recovering the polypeptide.

### Compositions

The present invention further relates to cleaning compositions comprising at least one DNase variant according to the invention and at least one cleaning adjunct ingredient. The cleaning composition may be used for improving deep-cleaning effect, including but not limited to deep cleaning of an item, for preventing and/or reducing the stickiness of an item, for pretreating stains on the item, for preventing and/or reducing redeposition of soil during a wash cycle, for preventing and/or reducing adherence of soil to an item, for maintaining or improving the whiteness of an item and for preventing and/or reducing malodor from an item. The DNase variants of the invention are useful in powder and liquid cleaning compositions as well as in e.g. single unit dose compositions.

The composition may contain one or more cleaning adjunct ingredient selected from the group consisting of surfactants, builders, flocculating aid, chelating agents, dye transfer inhibitors, enzymes, enzyme stabilizers, enzyme inhibitors, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, builders and co-builders, fabric hueing agents, anti-foaming agents, dispersants, processing aids, and/or pigments.

The cleaning composition will typically contain a surfactant and normally other cleaning adjunct ingredients such as a builder or a clay/soil removal/anti-redeposition agent.

The cleaning adjunct ingredient may be one or more enzymes other than a DNase. The one or more enzymes may be selected from the group consisting of proteases, amylases, lipases, cutinases, cellulases, endoglucanases, xyloglucanases, pectinases, pectin lyases, xanthanases, peroxidaes, haloperoxygenases, catalases and mannanases. Specific enzymes suitable for the detergent compositions of the invention are described below.

The cleaning composition may be formulated in any suitable form, such as a bar, a homogenous tablet, a tablet having two or more layers, a pouch having one or more compartments, a regular or compact powder, a granule, a paste, a gel, or a regular, compact or concentrated liquid. The cleaning composition can thus e.g. be a liquid detergent or a powder or granular detergent, optionally in "concentrated" or "compact" form. It may also be in the form of a single unit dose composition.

The DNase can be included in the cleaning composition of the present invention at a level of from 0.01 to 1000 ppm, from 1 ppm to 1000 ppm, from 10 ppm to 1000 ppm, from 50 ppm to 1000 ppm, from 100 ppm to 1000 ppm, from 150 ppm to 1000 ppm, from 200 ppm to 1000 ppm, from 250 ppm to 1000 ppm, from 250 ppm to 750 ppm, or from 250 ppm to 500 ppm.

In some aspects, the detergent composition is a liquid or powder laundry detergent, suitable for e.g. washing at high temperature and/or pH, such as at or above 40°C and/or at or above pH 8. In some aspects, the detergent composition is a liquid or powder laundry detergent, suitable for e.g. washing at low temperature and/or pH, such as at or below 20°C and/or pH 6. The detergent may also be formulated as a unit dose detergent and/or compact detergent optionally with minimum or no water. The detergent may also be a dish wash detergent. The laundry and dish wash detergents may be phosphate-free.

### Surfactants

A surfactant may be selected among nonionic, anionic and/or amphoteric surfactants as described above, preferably anionic or nonionic surfactants but also amphoteric surfactants may be used. In general, bleach-stable surfactants are preferred. Preferred anionic surfactants are sulphate surfactants and in particular alkyl ether sulphates, especially C-9-15 alcohol ethersulfates, C12-15 primary alcohol ethoxylate, C8-C16 ester sulphates and C10-C14 ester sulphates, such as mono dodecyl ester sulphates Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or salt of fatty acids (soap), and combinations thereof.

The anionic surfactants are preferably added to the detergent in the form of salts. Suitable cations in these salts are alkali metal ions, such as sodium, potassium and lithium and ammonium salts, for example (2-hydroxyethyl) ammonium, bis(2-hydroxyethyl) ammonium and tris(2-hydroxyethyl) ammonium salts. Non-limiting examples of nonionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxyalkyl fatty acid amides, or N-acyl N-alkyl derivatives of glucosamine (glucamides, GA, or fatty acid glucamides, FAGA), as well as products available under the trade names SPAN and TWEEN, and combinations thereof. Commercially available nonionic surfactants include Plurafac ^{™}, Lutensol^{™} and Pluronic^{™} from BASF, Dehypon^{™} series from Cognis and Genapol^{™} series from Clariant.

### Builders

A builder is preferably selected among phosphates, sodium citrate builders, sodium carbonate, sodium silicate, sodium aluminosilicate (zeolite). Suitable builders are alkali metal or ammonium phosphates, polyphosphates, phosphonates, polyphosphates, carbonates, bicarbonates, borates, citrates, and polycarboxylates. Citrate builders, e.g., citric acid and soluble salts thereof (particularly sodium salt), are polycarboxylate builders. Citrates can be used in combination with zeolite, silicates like the BRITESIL types, and/or layered silicate builders. The builder is preferably added in an amount of about 0-65% by weight, such as about 5% to about 50% by weight. In a laundry detergent, the level of builder is typically about 40-65% by weight, particularly about 50-65% by weight, particularly from 20% to 50% by weight. The builder and/or co-builder may particularly be a chelating agent that forms water-soluble complexes with Ca and Mg. Any builder and/or co-builder known in the art for use in cleaning detergents may be utilized. Non-limiting examples of builders include zeolites, diphosphates (pyrophosphates), triphosphates such as sodium triphosphate (STP or STPP), carbonates such as sodium carbonate, soluble silicates such as sodium metasilicate, layered silicates (e.g., SKS-6 from Hoechst), and (carboxymethyl)inulin (CMI), and combinations thereof. Further non-limiting examples of builders include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-N,N'-disuccinic acid (EDDS), methylglycine-N,N-diacetic acid (MGDA), glutamic acid-N,N-diacetic acid (GLDA), 1-hydroxyethane-1,1-diphosphonic acid, N-(2-hydroxyethyl)iminodiacetic acid (EDG), aspartic acid-N-monoacetic acid (ASMA), aspartic acid-N,N-diacetic acid (ASDA), aspartic acid-N-monopropionic acid (ASMP), iminodisuccinic acid (IDA), N-(sulfomethyl)aspartic acid (SMAS), N-(2-sulfoethyl)-aspartic acid (SEAS), N-(sulfomethylglutamic acid (SMGL), N-(2-sulfoethyl)-glutamic acid (SEGL), N-methyliminodiacetic acid (MIDA), serine-N,N-diacetic acid (SEDA), isoserine-N,N-diacetic acid (ISDA), phenylalanine-N,N-diacetic acid (PHDA), anthranilic acid-N,N-diacetic acid (ANDA), sulfanilic acid-N,N-diacetic acid (SLDA) , taurine-N,N-diacetic acid (TUDA) and N'-(2-hydroxyethyl)ethylenediamine-N,N,N'-triacetic acid (HEDTA), diethanolglycine (DEG), and combinations and salts thereof. Phosphonates suitable for use herein include 1-hydroxyethane-1,1-diphosphonic acid (HEDP), ethylenediaminetetrakis (methylenephosphonicacid) (EDTMPA), diethylenetriaminepentakis (methylenephosphonic acid) (DTMPA or DTPMPA or DTPMP), nitrilotris (methylenephosphonic acid) (ATMP or NTMP), 2-phosphonobutane-1,2,4-tricarboxylic acid (PBTC), hexamethylenediaminetetrakis (methylenephosphonic acid) (HDTMP). The composition may also contain 0-50% by weight, such as about 5% to about 30%, of a detergent co-builder. The detergent composition may include a co-builder alone, or in combination with a builder, for example a zeolite builder. Non-limiting examples of co-builders include homopolymers of polyacrylates or copolymers thereof, such as poly (acrylic acid) (PAA) or copoly (acrylic acid/maleic acid) (PAA/PMA) or polyaspartic acid. Further exemplary builders and/or co-builders are described in, e.g., WO 09/102854, US 5977053. In some aspects, the builder is a non-phosphorus based builder such as citric acid and/or methylglycine-N, N-diacetic acid (MGDA) and/or glutamic-N, N-diacetic acid (GLDA) and/or salts thereof. The liquid composition may also be phosphate free in that instance the preferred builders includes citrate and/or methylglycine-N, N-diacetic acid (MGDA) and/or glutamic-N, N-diacetic acid (GLDA) and / or salts thereof.

### Bleach components

The cleaning composition may contain 0-30% by weight, such as about 1% to about 20%, of a bleaching system. Any bleaching system comprising components known in the art for use in cleaning detergents may be utilized. Suitable bleaching system components include sources of hydrogen peroxide; sources of peracids; and bleach catalysts or boosters.

Sources of hydrogen peroxide: Suitable sources of hydrogen peroxide are inorganic persalts, including alkali metal salts such as sodium percarbonate and sodium perborates (usually mono- or tetrahydrate), and hydrogen peroxide-urea (1/1).

Sources of peracids: Peracids may be (a) incorporated directly as preformed peracids or (b) formed in situ in the wash liquor from hydrogen peroxide and a bleach activator (perhydrolysis) or (c) formed in situ in the wash liquor from hydrogen peroxide and a perhydrolase and a suitable substrate for the latter, e.g., an ester.
a) Suitable preformed peracids include, but are not limited to, peroxycarboxylic acids such as peroxybenzoic acid and its ring-substituted derivatives, peroxy-α-naphthoic acid, peroxyphthalic acid, peroxylauric acid, peroxystearic acid, ε-phthalimidoperoxycaproic acid [phthalimidoperoxyhexanoic acid (PAP)], and o-carboxybenzamidoperoxycaproic acid; aliphatic and aromatic diperoxydicarboxylic acids such as diperoxydodecanedioic acid, diperoxyazelaic acid, diperoxysebacic acid, diperoxybrassylic acid, 2-decyldiperoxybutanedioic acid, and diperoxyphthalic, -isophthalic and -terephthalic acids; perimidic acids; peroxymonosulfuric acid; peroxydisulfuric acid; peroxyphosphoric acid; peroxysilicic acid; and mixtures of said compounds. It is understood that the peracids mentioned may in some cases be best added as suitable salts, such as alkali metal salts (e.g., Oxone^{®}) or alkaline earth-metal salts.
b) Suitable bleach activators include those belonging to the class of esters, amides, imides, nitriles or anhydrides and, where applicable, salts thereof. Suitable examples are tetraacetylethylenediamine (TAED), sodium 4-[(3,5,5-trimethylhexanoyl)oxy]benzene-1-sulfonate (ISONOBS), sodium 4-(dodecanoyloxy)benzene-1-sulfonate (LOBS), sodium 4-(decanoyloxy)benzene-1-sulfonate, 4-(decanoyloxy)benzoic acid (DOBA), sodium 4-(nonanoyloxy)benzene-1-sulfonate (NOBS), and/or those disclosed in WO98/17767. A particular family of bleach activators of interest was disclosed in EP624154 and particularly preferred in that family is acetyl triethyl citrate (ATC). ATC or a short chain triglyceride like triacetin has the advantage that they are environmentally friendly. Furthermore, acetyl triethyl citrate and triacetin have good hydrolytical stability in the product upon storage and are efficient bleach activators. Finally, ATC is multifunctional, as the citrate released in the perhydrolysis reaction may function as a builder.

Bleach catalysts and boosters: The bleaching system may also include a bleach catalyst or booster. Some non-limiting examples of bleach catalysts that may be used in the compositions of the present invention include manganese oxalate, manganese acetate, mangarese-collagen, cobalt-amine catalysts and manganese triazacyclononane (MnTACN) catalysts; particularly preferred are complexes of manganese with 1,4,7-trimethyl-1,4,7-triazacyclononane (Me3-TACN) or 1,2,4,7-tetramethyl-1,4,7-triazacyclononane (Me4-TACN), in particular Me3-TACN, such as the dinuclear manganese complex [(Me3-TACN)Mn(O)3Mn(Me3-TACN)](PF6)2, and [2,2',2"-nitrilotris(ethane-1,2-diylazanylylidene-κN-methanylylidene)triphenolato-κ3O]manganese(III). The bleach catalysts may also be other metal compounds, such as iron or cobalt complexes. Other suitable bleach catalysts are acylhydrazone catalysts such as those disclosed in US 2014/0323381.

In some aspects, where a source of a peracid is included, an organic bleach catalyst or bleach booster may be used having one of the following formulae:
(i) 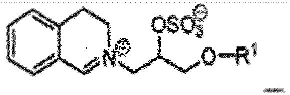
(ii) 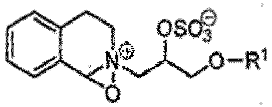
(iii) and mixtures thereof; wherein each R1 is independently a branched alkyl group containing from 9 to 24 carbons or linear alkyl group containing from 11 to 24 carbons, preferably each R1 is independently a branched alkyl group containing from 9 to 18 carbons or linear alkyl group containing from 11 to 18 carbons, more preferably each R1 is independently selected from the group consisting of 2-propylheptyl, 2-butyloctyl, 2-pentylnonyl, 2-hexyldecyl, dodecyl, tetradecyl, hexadecyl, octadecyl, isononyl, isodecyl, isotridecyl and isopentadecyl.

Other exemplary bleaching systems are described, e.g. in WO 2007/087258, WO 2007/087244, WO 2007/087259, EP 1867708 (Vitamin K) and WO 2007/087242. Suitable photobleaches may for example be sulfonated zinc or aluminium phthalocyanines.

The choice of detergent components may include, for textile care, the consideration of the type of textile to be cleaned, the type and/or degree of soiling, the temperature at which cleaning is to take place, and the formulation of the detergent product. Although components mentioned below are categorized by general header according to a functionality, this is not to be construed as a limitation, as a component may comprise additional functionalities as will be appreciated by the skilled artisan, including the exemplary non-limiting components shown in below.

### Hydrotropes

The detergent composition may contain 0-10% by weight, for example 0-5% by weight, such as about 0.5 to about 5%, or about 3% to about 5%, of a hydrotrope. Any hydrotrope known in the art for use in detergents may be utilized. Non-limiting examples of hydrotropes include sodium benzenesulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

### Polymers

The detergent composition may contain 0-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fibre protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-*N*-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, e.g., WO 2006/130575. Salts of the above-mentioned polymers are also contemplated.

### Fabric hueing agents

The detergent composition of the present invention may also include fabric hueing agents such as dyes or pigments, which when formulated in detergent compositions can deposit onto a fabric when said fabric is contacted with a wash liquor comprising said detergent compositions and thus altering the tint of said fabric through absorption/reflection of visible light. Fluorescent whitening agents emit at least some visible light. In contrast, fabric hueing agents alter the tint of a surface as they absorb at least a portion of the visible light spectrum. Suitable fabric hueing agents include dyes and dye-clay conjugates, and may also include pigments. Suitable dyes include small molecule dyes and polymeric dyes. Suitable small molecule dyes include small molecule dyes selected from the group consisting of dyes falling into the Colour Index (C.I.) classifications of Direct Blue, Direct Red, Direct Violet, Acid Blue, Acid Red, Acid Violet, Basic Blue, Basic Violet and Basic Red, or mixtures thereof, for example as described in WO 2005/03274, WO 2005/03275, WO 2005/03276 and EP 1876226 (hereby incorporated by reference). The detergent composition preferably comprises from about 0.00003 wt% to about 0.2 wt%, from about 0.00008 wt% to about 0.05 wt%, or even from about 0.0001 wt% to about 0.04 wt% fabric hueing agent. The composition may comprise from 0.0001 wt% to 0.2 wt% fabric hueing agent, this may be especially preferred when the composition is in the form of a unit dose pouch. Suitable hueing agents are also disclosed in, e.g. WO 2007/087257 and WO 2007/087243.

### Enzymes

The detergent composition may comprise one or more additional enzymes such as a protease, lipase, cutinase, amylase, carbohydrase, cellulase, pectinase, mannanase, arabinase, galactanase, xylanase, hexosaminidase, oxidase, *e.g*., a laccase, and/or peroxidase.

In general, the properties of the selected enzyme(s) should be compatible with the selected detergent, (*i.e.,* pH-optimum, compatibility with other enzymatic and non-enzymatic ingredients, etc.), and the enzyme(s) should be present in effective amounts.

### Cellulases

Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having colour care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US 5,763,254, WO 95/24471, WO 98/12307 and WO 99/001544.

Other cellulases are endo-beta-1,4-glucanase enzyme having a sequence of at least 97% identity to the amino acid sequence of position 1 to position 773 of SEQ ID NO: 2 of WO 2002/099091 or a family 44 xyloglucanase, which a xyloglucanase enzyme having a sequence of at least 60% identity to positions 40-559 of SEQ ID NO: 2 of WO 2001/062903.

Commercially available cellulases include Celluzyme^{™}, and Carezyme^{™} (Novozymes A/S) Carezyme Premium^{™} (Novozymes A/S), Celluclean ^{™} (Novozymes A/S), Celluclean Classic^{™} (Novozymes A/S), Cellusoft^{™} (Novozymes A/S), Whitezyme^{™} (Novozymes A/S), Clazinase^{™}, and Puradax HA^{™} (Genencor International Inc.), and KAC-500(B)^{™} (Kao Corporation).

### Proteases

Suitable proteases may be of any origin, but are preferably of bacterial or fungal origin, optionally in the form of protein engineered or chemically modified mutants. The protease may be an alkaline protease, such as a serine protease or a metalloprotease. A serine protease may for example be of the S1 family, such as trypsin, or the S8 family such as a subtilisin. A metalloprotease may for example be a thermolysin, e.g. from the M4 family, or another metalloprotease such as those from the M5, M7 or M35 families.

The term "subtilases" refers to a sub-group of serine proteases according to Siezen et al., Protein Eng. 4 (1991) 719-737 and Siezen et al., Protein Sci. 6 (1997) 501-523. Serine proteases are a subgroup of proteases characterized by having a serine in the active site, which forms a covalent adduct with the substrate. The subtilases may be divided into six subdivisions, the Subtilisin family, the Thermitase family, the Proteinase K family, the Lantibiotic peptidase family, the Kexin family and the Pyrolysin family.

Although proteases suitable for detergent use may be obtained from a variety of organisms, including fungi such as *Aspergillus,* detergent proteases have generally been obtained from bacteria and in particular from *Bacillus.* Examples of *Bacillus* species from which subtilases have been derived include *Bacillus lentus, Bacillus alkalophilus, Bacillus subtilis, Bacillus amyloliquefaciens, Bacillus licheniformis, Bacillus pumilus* and *Bacillus gibsonii.* Particular subtilisins include *subtilisin lentus, subtilisin* Novo, *subtilisin* Carlsberg, *subtilisin* BPN', *subtilisin* 309, *subtilisin* 147 and *subtilisin* 168 and e.g. protease PD138 (described in WO 93/18140). Other useful proteases are e.g. those described in WO 01/16285 and WO 02/16547.

Examples of trypsin-like proteases include the *Fusarium* protease described in WO 94/25583 and WO 2005/040372, and the chymotrypsin proteases derived from *Cellumonas* described in WO 2005/052161 and WO 2005/052146.

Examples of metalloproteases include the neutral metalloproteases described in WO 2007/044993 such as those derived from *Bacillus amyloliquefaciens,* as well as e.g. the metalloproteases described in WO 2015/158723 and WO 2016/075078.

Examples of useful proteases are the protease variants described in WO 89/06279 WO 92/19729, WO 96/34946, WO 98/20115, WO 98/20116, WO 99/11768, WO 01/44452, WO 03/006602, WO 2004/003186, WO 2004/041979, WO 2007/006305, WO 2011/036263, WO 2014/207227, WO 2016/087617 and WO 2016/174234. Preferred protease variants may, for example, comprise one or more of the mutations selected from the group consisting of: S3T, V4I, S9R, S9E, A15T, S24G, S24R, K27R, N42R, S55P, G59E, G59D, N60D, N60E, V66A, N74D, S85R, A96S, S97G, S97D, S97A, S97SD, S99E, S99D, S99G, S99M, S99N, S99R, S99H, S101A, V102I, V102Y, V102N, S104A, G116V, G116R, H118D, H118N, A120S, S126L, P127Q, S128A, S154D, A156E, G157D, G157P, S158E, Y161A, R164S, Q176E, N179E, S182E, Q185N, A188P, G189E, V193M, N198D, V199I, Q200L, Y203W, S206G, L211Q, L211D, N212D, N212S, M216S, A226V, K229L, Q230H, Q239R, N246K, S253D, N255W, N255D, N255E, L256E, L256D T268A and R269H, wherein position numbers correspond to positions of the *Bacillus lentus* protease shown in SEQ ID NO: 1 of WO 2016/001449. Protease variants having one or more of these mutations are preferably variants of the *Bacillus lentus* protease (Savinase^{®}, also known as subtilisin 309) shown in SEQ ID NO: 1 of WO 2016/001449 or of the *Bacillus amyloliquefaciens* protease (BPN') shown in SEQ ID NO: 2 of WO 2016/001449. Such protease variants preferably have at least 80% sequence identity to SEQ ID NO: 1 or to SEQ ID NO: 2 of WO 2016/001449.

Another protease of interest is the alkaline protease from *Bacillus lentus* DSM 5483, as described for example in WO 91/02792, and variants thereof which are described for example in WO 92/21760, WO 95/23221, EP 1921147, EP 1921148 and WO 2016/096711.

The protease may alternatively be a variant of the TY145 protease having SEQ ID NO: 1 of WO 2004/067737, for example a variant comprising a substitution at one or more positions corresponding to positions 27, 109, 111, 171, 173, 174, 175, 180, 182, 184, 198, 199 and 297 of SEQ ID NO: 1 of WO 2004/067737, wherein said protease variant has a sequence identity of at least 75% but less than 100% to SEQ ID NO: 1 of WO 2004/067737. TY145 variants of interest are described in e.g. WO 2015/014790, WO 2015/014803, WO 2015/014804, WO 2016/097350, WO 2016/097352, WO 2016/097357 and WO 2016/097354.

Suitable commercially available protease enzymes include those sold under the trade names Alcalase^{®}, Duralase^{™}, Durazym^{™}, Relase^{®}, Relase^{®} Ultra, Savinase^{®}, Savinase^{®} Ultra, Primase^{™}, Polarzyme^{®}, Kannase^{®}, Liquanase^{®}, Liquanase^{®} Ultra, Ovozyme^{®}, Coronase^{®}, Coronase^{®} Ultra, Blaze^{®}, Blaze Evity^{®} 100T, Blaze Evity^{®} 125T, Blaze Evity^{®} 150T, Blaze Evity^{®} 200T, Neutrase^{®}, Everlase^{®}, Esperase^{®}, Progress^{®} Uno, Progress^{®} In and Progress^{®} Excel (Novozymes A/S), those sold under the tradename Maxatase^{™}, Maxacal^{™}, Maxapem^{®}, Purafect^{®} Ox, Purafect^{®} OxP, Puramax^{®}, FN2^{™}, FN3^{™}, FN4^{ex™}, Excellase^{®}, Excellenz^{™} P1000, Excellenz^{™} P1250, Eraser^{™}, Preferenz^{®} P100, Purafect Prime, Preferenz P110^{™}, Effectenz P1000^{™}, Purafect^{®}, Effectenz P1050^{™}, Purafect^{®} Ox, Effectenz^{™} P2000, Purafast^{™}, Properase^{®}, Opticlean^{™} and Optimase^{®} (Danisco/DuPont), BLAP (sequence shown in Figure 29 of US 5352604) and variants hereof (Henkel AG), and KAP (*Bacillus alkalophilus* subtilisin) from Kao.

### Lipases and Cutinases:

Suitable lipases and cutinases include those of bacterial or fungal origin. Chemically modified or protein engineered mutant enzymes are included. Examples include lipase from *Thermomyces,* e.g. from *T. lanuginosus* (previously named *Humicola lanuginosa*) as described in EP258068 and EP305216, cutinase from *Humicola,* e.g. *H. insolens* (WO96/13580), lipase from strains of *Pseudomonas* (some of these now renamed to *Burkholderia*)*,* e.g. *P. alcaligenes* or *P*. *pseudoalcaligenes* (EP218272), *P*. *cepacia* (EP331376), *P*. *sp.* strain SD705 (WO95/06720 & WO96/27002), *P*. *wisconsinensis* (WO96/12012), GDSL-type *Streptomyces* lipases (WO10/065455), cutinase from *Magnaporthe grisea* (WO10/107560), cutinase from *Pseudomonas mendocina* (US5,389,536), lipase from *Thermobifida fusca* (WO11/084412), *Geobacillus stearothermophilus* lipase (WO11/084417), lipase from *Bacillus subtilis* (WO11/084599), and lipase from *Streptomyces griseus* (WO11/150157) and *S*. *pristinaespiralis* (WO12/137147).

Other examples are lipase variants such as those described in EP407225, WO92/05249, WO94/01541, WO94/25578, WO95/14783, WO95/30744, WO95/35381, WO95/22615, WO96/00292, WO97/04079, WO97/07202, WO00/34450, WO00/60063, WO01/92502, WO07/87508 and WO09/109500.

Preferred commercial lipase products include Lipolase^{™}, Lipex^{™}; Lipolex^{™} and Lipoclean^{™} (Novozymes A/S), Lumafast (originally from Genencor) and Lipomax (originally from Gist-Brocades).

Still other examples are lipases sometimes referred to as acyltransferases or perhydrolases, e.g. acyltransferases with homology to *Candida antarctica* lipase A (WO10/111143), acyltransferase from *Mycobacterium smegmatis* (WO05/56782), perhydrolases from the CE 7 family (WO09/67279), and variants of the *M. smegmatis* perhydrolase in particular the S54V variant used in the commercial product Gentle Power Bleach from Huntsman Textile Effects Pte Ltd (WO10/100028).

### Amylases

Suitable amylases which can be used together with the DNases of the invention may be an alpha-amylase or a glucoamylase and may be of bacterial or fungal origin. Chemically modified or protein engineered mutants are included. Amylases include, for example, alpha-amylases obtained from *Bacillus, e.g.,* a special strain of *Bacillus licheniformis,* described in more detail in GB 1,296,839.

Suitable amylases include amylases having SEQ ID NO: 2 in WO 95/10603 or variants having 90% sequence identity to SEQ ID NO: 1 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181 and 182 and a substitution in position 193.

Other amylases which are suitable are hybrid alpha-amylase comprising residues 1-33 of the alpha-amylase obtained from *B*. *amyloliquefaciens* shown in SEQ ID NO: 6 of WO 2006/066594 and residues 36-483 of the *B*. *licheniformis* alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, I201, A209 and Q264. Most preferred variants of the hybrid alpha-amylase comprising residues 1-33 of the alpha-amylase obtained from *B. amyloliquefaciens* shown in SEQ ID NO: 6 of WO 2006/066594 and residues 36-483 of SEQ ID NO: 4 are those having the substitutions:
M197T;
H156Y+A181T+N190F+A209V+Q264S; or
G48A+T49I+G107A+H156Y+A181T+ N190F+1201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476, using SEQ ID NO:. 2 of WO 96/023873 for numbering. More preferred variants are those having a deletion in two positions selected from 181, 182, 183 and 184, such as 181 and 182, 182 and 183, or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:
N128C+K178L+T182G+Y305R+G475K;
N128C+K178L+T182G+F202Y+Y305R+D319T+G475K;
S125A+N128C+K178L+T182G+Y305R+G475K; or
S125A+N128C+T131I+T165I+K178L+T182G+Y305R+G475K wherein the variants are C-terminally truncated and optionally further comprises a substitution at position 243 and/or a deletion at position 180 and/or position 181.

Other suitable amylases are the alpha-amylase having SEQ ID NO: 12 in WO 01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particular preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO 2011/098531, WO 2013/001078 and WO 2013/001087.

Commercially available amylases include Duramyl^{™}, Termamyl^{™}, Fungamyl^{™}, Stainzyme^{™}, Stainzyme Plus^{™}, Natalase^{™}, Liquozyme X and BAN^{™} (from Novozymes A/S), and Rapidase^{™}, Purastar^{™}/Effectenz^{™}, Powerase and Preferenz S100 (from Genencor International Inc./DuPont).

### Hexosaminidases

Detergent compositions comprising a DNase of the invention may also include one or more hexosaminidases. The term hexosaminidase includes "dispersin" and the abbreviation "Dsp", which means a polypeptide having hexosaminidase activity, EC 3.2.1.-, that catalyzes the hydrolysis of β-1,6-glycosidic linkages of N-acetyl-glucosamine polymers found e.g. in biofilm. The term hexosaminidase includes polypeptides having N-acetylglucosaminidase activity and β-N-acetylglucosaminidase activity.

A polypeptide having hexosaminidase activity may be obtained from microorganisms of any genus, in particular from bacteria or fungi. Preferably the hexosaminidase, e.g. a dispersin, is obtained from *Terribacillus, Curtobacterium, Aggregatibacter, Haemophilus* or *Actinobacillus,* preferably *Terribacillus.* The hexosaminidase may also be a variant of a polypeptide obtained from any of these or other organisms.

Suitable hexosaminidases include those disclosed in WO2017186936, WO2017186937, WO2017186943, WO2017207770, WO2018184873, WO2019086520, WO2019086528, WO2019086530, WO2019086532, WO2019086521, WO2019086526, WO2020002604, WO2020002608, WO2020007863, WO2020007875, WO2020008024, WO2020070063, WO2020070249, WO2020088957, WO2020088958 and WO2020207944.

### Peroxidases/Oxidases

A peroxidase may be comprised by the enzyme classification EC 1.11.1.7, as set out by the Nomenclature Committee of the International Union of Biochemistry and Molecular Biology (IUBMB), or any fragment obtained therefrom, exhibiting peroxidase activity. Suitable peroxidases include those of plant, bacterial or fungal origin. Chemically modified or protein engineered mutants are included. Examples of useful peroxidases include peroxidases from *Coprinopsis, e.g.,* from *C*. *cinerea* (EP 179,486), and variants thereof as those described in WO 93/24618, WO 95/10602, and WO 98/15257. A peroxidase may also include a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions. In an aspect, the haloperoxidase is a chloroperoxidase. Preferably, the haloperoxidase is a vanadium haloperoxidase, *i.e.,* a vanadate-containing haloperoxidase. In a preferred method the vanadate-containing haloperoxidase is combined with a source of chloride ion. Haloperoxidases have been isolated from many different fungi, in particular from the fungus group dematiaceous hyphomycetes, such as *Caldariomyces, e.g., C*. *fumago, Alternaria, Curvularia, e.g., C*. *verruculosa* and *C*. *inaequalis, Drechslera, Ulocladium* and *Botrytis.* Haloperoxidases have also been isolated from bacteria such as *Pseudomonas, e.g., P. pyrrocinia* and *Streptomyces, e.g., S. aureofaciens.* In a preferred aspect, the haloperoxidase is derivable from *Curvularia* sp., in particular *Curvularia verruculosa* or *Curvularia inaequalis,* such as *C*. *inaequalis* CBS 102.42 as described in WO 95/27046; or *C*. *verruculosa* CBS 147.63 or *C*. *verruculosa* CBS 444.70 as described in WO 97/04102; or from *Drechslera hartlebii* as described in WO 01/79459, *Dendryphiella salina* as described in WO 01/79458, *Phaeotrichoconis crotalarie* as described in WO 01/79461, or *Geniculosporium* sp. as described in WO 01/79460.

An oxidase includes any laccase enzyme comprised by the enzyme classification EC 1.10.3.2, or any fragment obtained therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5).

Preferred laccase enzymes are enzymes of microbial origin. The enzymes may be obtained from plants, bacteria or fungi (including filamentous fungi and yeasts).

Suitable examples from fungi include a laccase derivable from a strain of *Bacillus, Neurospora, e.g., N. crassa, Podospora, Botrytis, Collybia, Fomes, Lentinus, Pleurotus, Trametes, e.g., T. villosa* and *T. versicolor, Rhizoctonia, e.g., R. solani, Coprinopsis, e.g., C*. *cinerea, C. comatus, C. friesii,* and *C*. *plicatilis, Psathyrella, e.g., P. condelleana, Panaeolus, e.g., P. papilionaceus, Myceliophthora, e.g., M. thermophila, Schytalidium, e.g., S. thermophilum, Polyporus, e.g., P. pinsitus, Phlebia, e.g., P. radiata* (WO 92/01046), or *Coriolus, e.g., C. hirsutus* (JP 2238885).

Suitable examples from bacteria include a laccase derivable from a strain of *Bacillus.*

A laccase obtained from *Coprinopsis* or *Myceliophthora* is preferred; a laccase obtained from *Coprinopsis cinerea,* as disclosed in WO 97/08325; or from *Myceliophthora thermophila,* as disclosed in WO 95/33836.

The detergent enzyme(s) may be included in a detergent composition by adding separate additives containing one or more enzymes, or by adding a combined additive comprising these enzymes. A detergent additive of the invention, *i.e.,* a separate additive or a combined additive, can be formulated, for example, as a granulate, liquid, slurry, etc. Preferred detergent additive formulations are granulates, non-dusting granulates, liquids, stabilized liquids, or slurries.

Non-dusting granulates may be produced, *e.g.* as disclosed in US 4,106,991 and 4,661,452 and may optionally be coated by methods known in the art. Examples of waxy coating materials are poly (ethylene oxide) products (polyethyleneglycol, PEG) with mean molar weights of 1000 to 20000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591. Liquid enzyme preparations may, for instance, be stabilized by adding a polyol such as propylene glycol, a sugar or sugar alcohol, lactic acid or boric acid according to established methods. Protected enzymes may be prepared according to the method disclosed in EP 238,216.

### Dispersants

The detergent compositions of the present invention can also contain dispersants. Powdered detergents may comprise dispersants. Suitable water-soluble organic materials include the homo- or co-polymeric acids or their salts, in which the polycarboxylic acid comprises at least two carboxyl radicals separated from each other by not more than two carbon atoms. Suitable dispersants are for example described in Powdered Detergents, Surfactant science series volume 71, Marcel Dekker, Inc.

### Dye Transfer Inhibiting Agents

The cleaning compositions of the present invention may also include one or more dye transfer inhibiting agents. Suitable polymeric dye transfer inhibiting agents include, but are not limited to, polyvinylpyrrolidone polymers, polyamine *N*-oxide polymers, copolymers of *N-*vinylpyrrolidone and *N*-vinylimidazole, polyvinyloxazolidones and polyvinylimidazoles or mixtures thereof. When present in a subject composition, the dye transfer inhibiting agents may be present at levels from about 0.0001 % to about 10%, from about 0.01% to about 5% or even from about 0.1% to about 3% by weight of the composition.

### Fluorescent whitening agent

The detergent composition may preferably also contain additional components that may tint articles being cleaned, such as fluorescent whitening agent or optical brighteners. Where present the brightener is preferably at a level of about 0.01% to about 0.5%. Any fluorescent whitening agent suitable for use in a laundry detergent composition may be used in the composition of the present invention. The most commonly used fluorescent whitening agents are those belonging to the classes of diaminostilbene-sulfonic acid derivatives, diarylpyrazoline derivatives and bisphenyl-distyryl derivatives. Examples of the diaminostilbene-sulfonic acid derivative type of fluorescent whitening agents include the sodium salts of: 4,4'-bis-(2-diethanolamino-4-anilino-s-triazin-6-ylamino) stilbene-2,2'-disulfonate, 4,4'-bis-(2,4-dianilino-s-triazin-6-ylamino) stilbene-2.2'-disulfonate, 4,4'-bis-(2-anilino-4-(*N*-methyl-*N*-2-hydroxy-ethylamino)-s-triazin-6-ylamino) stilbene-2,2'-disulfonate, 4,4'-bis-(4-phenyl-1,2,3-triazol-2-yl)stilbene-2,2'-disulfonate and sodium 5-(2*H*-naphtho[1,2-*d*][1,2,3]triazol-2-yl)-2-[(*E*)-2-phenylvinyl]benzenesulfonate. Preferred fluorescent whitening agents are Tinopal DMS and Tinopal CBS available from Ciba-Geigy AG, Basel, Switzerland. Tinopal DMS is the disodium salt of 4,4'-bis-(2-morpholino-4-anilino-s-triazin-6-ylamino) stilbene-2,2'-disulfonate. Tinopal CBS is the disodium salt of 2,2'-bis-(phenyl-styryl)-disulfonate. Also preferred are fluorescent whitening agents is the commercially available Parawhite KX, supplied by Paramount Minerals and Chemicals, Mumbai, India. Tinopal CBS-X is a 4.4'-bis-(sulfostyryl)-biphenyl disodium salt also known as Disodium Distyrylbiphenyl Disulfonate. Other fluorescers suitable for use in the invention include the 1-3-diaryl pyrazolines and the 7-alkylaminocoumarins.

Suitable fluorescent brightener levels include lower levels of from about 0.01, from 0.05, from about 0.1 or even from about 0.2 wt % to upper levels of 0.5 or even 0.75 wt%.

### Soil release polymers

The detergent compositions may also include one or more soil release polymers which aid the removal of soils from fabrics such as cotton and polyester based fabrics, the removal of hydrophobic soils from polyester based fabrics. The soil release polymers may for example be nonionic or anionic terephthalte based polymers, polyvinyl caprolactam and related copolymers, vinyl graft copolymers, polyester polyamides see for example Chapter 7 in Powdered Detergents, Surfactant science series volume 71, Marcel Dekker, Inc. Another type of soil release polymers is amphiphilic alkoxylated grease cleaning polymers comprising a core structure and a plurality of alkoxylate groups attached to that core structure. The core structure may comprise a polyalkylenimine structure or a polyalkanolamine structure as described in detail in WO 2009/087523 (hereby incorporated by reference). Furthermore, random graft co-polymers are suitable soil release polymers. Suitable graft co-polymers are described in more detail in WO 2007/138054, WO 2006/108856 and WO 2006/113314 (hereby incorporated by reference). Other soil release polymers are substituted polysaccharide structures especially substituted cellulosic structures such as modified cellulose deriviatives such as those described in EP 1867808 or WO 2003/040279 (both are hereby incorporated by reference). Suitable cellulosic polymers include cellulose, cellulose ethers, cellulose esters, cellulose amides and mixtures thereof. Suitable cellulosic polymers include anionically modified cellulose, nonionically modified cellulose, cationically modified cellulose, zwitterionically modified cellulose, and mixtures thereof. Suitable cellulosic polymers include methyl cellulose, carboxy methyl cellulose, ethyl cellulose, hydroxyl ethyl cellulose, hydroxyl propyl methyl cellulose, ester carboxy methyl cellulose, and mixtures thereof.

### Anti-redeposition agents

The detergent compositions of the present invention may also include one or more anti-redeposition agents such as carboxymethylcellulose (CMC), polyvinyl alcohol (PVA), polyvinylpyrrolidone (PVP), polyoxyethylene and/or polyethyleneglycol (PEG), homopolymers of acrylic acid, copolymers of acrylic acid and maleic acid, and ethoxylated polyethyleneimines. The cellulose based polymers described under soil release polymers above may also function as anti-redeposition agents.

### Rheology Modifiers

The detergent compositions of the present invention may also include one or more rheology modifiers, structurants or thickeners, as distinct from viscosity reducing agents. The rheology modifiers are selected from the group consisting of non-polymeric crystalline, hydroxyfunctional materials, polymeric rheology modifiers which impart shear thinning characteristics to the aqueous liquid matrix of a liquid detergent composition. The rheology and viscosity of the detergent can be modified and adjusted by methods known in the art, for example as shown in EP 2169040.

Other suitable adjunct materials include, but are not limited to, anti-shrink agents, anti-wrinkling agents, bactericides, binders, carriers, dyes, enzyme stabilizers, fabric softeners, fillers, foam regulators, hydrotropes, perfumes, pigments, sod suppressors, solvents, and structurants for liquid detergents and/or structure elasticizing agents.

### Other materials

Any detergent components known in the art for use in the cleaning composition of the invention may also be utilized. Other optional detergent components include anti-corrosion agents, anti-shrink agents, anti-soil redeposition agents, anti-wrinkling agents, bactericides, binders, corrosion inhibitors, disintegrants/disintegration agents, dyes, enzyme stabilizers (including boric acid, borates, CMC, and/or polyols such as propylene glycol), fabric conditioners including clays, fillers/processing aids, fluorescent whitening agents/optical brighteners, foam boosters, foam (suds) regulators, perfumes, soil-suspending agents, softeners, suds suppressors, tarnish inhibitors, and wicking agents, either alone or in combination. Any ingredient known in the art for use in detergents may be utilized. The choice of such ingredients is well within the skill of the artisan.

### Formulation of detergent products

The detergent composition may be in any convenient form, e.g., a bar, a homogenous tablet, a tablet having two or more layers, a regular or compact powder, a granule, a paste, a gel, or a regular, compact or concentrated liquid. Other detergent formulation forms include single unit dose forms such as layered forms and pouches.

Pouches can be configured as single or multicompartments. They can be of any form, shape and material which is suitable for hold the composition, e.g. without allowing release of the composition from the pouch prior to water contact. The pouch is made from water soluble film which encloses an inner volume, which can be divided into compartments. Preferred films are polymeric materials, preferably polymers which are formed into a film or sheet. Preferred polymers, copolymers or derivates thereof are selected from polyacrylates and water soluble acrylate copolymers, methyl cellulose, carboxy methyl cellulose, sodium dextrin, ethyl cellulose, hydroxyethyl cellulose, hydroxypropyl methyl cellulose, maltodextrin, polymethacrylates, most preferably polyvinyl alcohol copolymers and hydroxypropyl methyl cellulose (HPMC). Preferably the level of polymer in a film such as is at least about 60%. Preferred average molecular weight will typically be about 20,000 to about 150,000. Films can also be of blend compositions comprising hydrolytically degradable and water soluble polymer blends such as polylactide and polyvinyl alcohol plus plasticisers such as glycerol, ethylene glycerol, propylene glycol, sorbitol and mixtures thereof. The pouches can comprise a solid laundry cleaning composition or part components and/or a liquid cleaning composition or part components separated by the water-soluble film. Compartments for liquid components can be different in composition than compartments containing solids; see e.g. US 2009/0011970 A1.

Detergent ingredients can be separated physically from each other by compartments in water dissolvable pouches or in different layers of tablets, thereby avoiding negative storage interaction between components. Different dissolution profiles of each of the compartments can also give rise to delayed dissolution of selected components in the wash solution.

A liquid or gel detergent which is not unit dosed may be aqueous, typically containing at least 20% by weight and up to 95% water, such as up to about 70% water, up to about 65% water, up to about 55% water, up to about 45% water, or up to about 35% water. Concentrated liquid detergents may have lower water contents, for example not more than about 30% or not more than about 20%, e.g. in the range of about 1% to about 20%, such as from about 2% to about 15%. Other types of liquids, including without limitation, alkanols, amines, diols, ethers and polyols may be included in an aqueous liquid or gel. An aqueous liquid or gel detergent may contain from 0-30% organic solvent. A liquid or gel detergent may alternatively be non-aqueous.

Liquid detergent compositions may be formulated to have a moderate pH of e.g. from about 6 to about 10, such as about pH 7, about pH 8 or about pH 9, or they may be formulated to have a higher pH of e.g. from about 10 to about 12, such as about pH 10, about pH 11 or about pH 12.

Unless indicated otherwise, the term "liquid" as used herein should be understood to encompass any kind of liquid detergent composition, for example concentrated liquids, gels, or the liquid or gel part of e.g. a pouch with one or more compartments.

### Granular detergent formulations

Enzymes in the form of granules, comprising an enzyme-containing core and optionally one or more coatings, are commonly used in granular (powder) detergents. Various methods for preparing the core are well-known in the art and include, for example, a) spray drying of a liquid enzyme-containing solution, b) production of layered products with an enzyme coated as a layer around a pre-formed inert core particle, e.g. using a fluid bed apparatus, c) absorbing an enzyme onto and/or into the surface of a pre-formed core, d) extrusion of an enzyme-containing paste, e) suspending an enzyme-containing powder in molten wax and atomization to result in prilled products, f) mixer granulation by adding an enzyme-containing liquid to a dry powder composition of granulation components, g) size reduction of enzyme-containing cores by milling or crushing of larger particles, pellets, etc., and h) fluid bed granulation. The enzyme-containing cores may be dried, e.g. using a fluid bed drier or other known methods for drying granules in the feed or enzyme industry, to result in a water content of typically 0.1 -10% w/w water.

The enzyme-containing cores are optionally provided with a coating to improve storage stability and/or to reduce dust formation. One type of coating that is often used for enzyme granulates for detergents is a salt coating, typically an inorganic salt coating, which may e.g. be applied as a solution of the salt using a fluid bed. Other coating materials that may be used are, for example, polyethylene glycol (PEG), methyl hydroxy-propyl cellulose (MHPC) and polyvinyl alcohol (PVA). The granules may contain more than one coating, for example a salt coating followed by an additional coating of a material such as PEG, MHPC or PVA.

For further information on enzyme granules and production thereof, see WO 2013/007594 as well as e.g. WO 2009/092699, EP 1705241, EP 1382668, WO 2007/001262, US 6,472,364, WO 2004/074419 and WO 2009/102854.

### Formulation of enzyme in co-granule

The DNase may be formulated as a granule for example as a co-granule that combines one or more enzymes. Each enzyme will then be present in more granules securing a more uniform distribution of enzymes in the detergent. This also reduces the physical segregation of different enzymes due to different particle sizes. Methods for producing multi-enzyme co-granulate for the detergent industry is disclosed in the IP.com disclosure IPCOM000200739D.

Another example of formulation of enzymes using co-granulates are disclosed in WO 2013/188331, which relates to a detergent composition comprising (a) a multi-enzyme co- granule; (b) less than 10 wt zeolite (anhydrous basis); and (c) less than 10 wt phosphate salt (anhydrous basis), wherein said enzyme co-granule comprises from 10 to 98 wt% moisture sink components and the composition additionally comprises from 20 to 80 wt% detergent moisture sink components. WO 2013/188331 also relates to a method of treating and/or cleaning a surface, preferably a fabric surface comprising the steps of (i) contacting said surface with the detergent composition as claimed and described herein aqueous wash liquor, (ii) rinsing and/or drying the surface.

The present invention is further described by the following examples that should not be construed as limiting the scope of the invention.

### EXAMPLES

### Materials and methods

### Assay I: Determination of DNase activity

DNase activity may be determined by using the DNaseAlert^{™} Kit (IDT Intergrated DNA Technologies, Belgium) according to the supplier's manual. See Example 2 below for an example of this assay.

### Assay II: Storage stability assay for purified DNase variants

The storage stability of purified DNase variants in detergent compositions, expressed as a half-life improvement factor relative to a reference DNase, may e.g. be determined as described in Example 3 below.

### Example 1: Construction and screening of variants

Site-directed variants of the DNase of SEQ ID NO: 1 comprising specific substitutions according to the invention were constructed. The variants were made by traditional cloning of DNA fragments (Sambrook et al., Molecular Cloning: A Laboratory Manual, 2nd Ed., Cold Spring Harbor, 1989) using PCR together with properly designed mutagenic oligonucleotides that introduced the desired mutations in the resulting sequence.

Mutagenic oligos were designed corresponding to the DNA sequence flanking the desired site(s) of mutation, separated by the DNA base pairs defining the insertions/deletions/- substitutions, and purchased from an oligo vendor such as Life Technologies. To test the DNase variants of the invention, mutated DNA comprising a variant of the invention was integrated into a competent *Bacillus subtilis* strain by homologous recombination. The transformations were grown for 1 hour in TB-Gly growth medium without antibiotic and then overnight in TB-Gly medium with 3 µg/ml chloramphenicol. After addition of glycerol to 25% v/v these were stored at -80°C. Replicates were grown for 3-4 days for DNase production in broth supplemented with chloramphenicol and trace elements: 50 µM FeCl3 , 20 µM CaCI2, 10 µM MnCI2, 10 µM ZnSO4 and 2 µM CuCl2.

### Example 2: Stability screening of DNase variants in supernatant

After growth, each supernatant sample was split into two identical samples by transferring 20 µl supernatant to two 96-well standard microtiter plates each containing 170 µl Tide Pods^{®} 3-in-1 detergent supplemented with 1% (v/v) of a liquid laundry protease, Progress^{®} Uno 101 L (Novozymes A/S), and 0.3% (w/v) sodium bisulfite. After shaking for 20 minutes at 7500 rpm in a microtiter plate shaker, one microtiter plate was incubated at 10°C (reference condition) for three days and the other microtiter plate was incubated in an incubator at 50°C for three days (stress condition). After three days, both sets of samples (the reference condition and the stressed condition) were diluted 20-fold in dilution buffer (50 mM Tris, HCI, 0.01% Tween20, pH 7.5) before assaying the activity using the DNAseAlert^{™} substrate solution (Integrated DNA Technologies, Belgium, part #11-04-02-04). 10 µl 20-fold diluted reference condition DNase sample and stress condition DNase sample were transferred to a new 384-well microtiter plate and 40 µl DNAseAlert^{™} assay solution was added (50 mM TrisHCl, pH 7.5, 5 mM MnCl₂, 0.01% Tween20, 10 µM DNAseAlert^{™} substrate). The fluorescence (excitation 536 nm, emission 556 nm) was read every 90 seconds for a total of 30 minutes. From the kinetic curves, the slope of the reference sample (activity under reference conditions) and the corresponding stress sample (activity under stress conditions) was determined by linear regression. The residual activity (RA) for each DNase variant and the reference DNase (SEQ ID NO: 1) was calculated as: slope (stress sample)/slope (reference sample).

The RA was used for calculation of half-lives, the half-life improvement factor being calculated as: stress time (minutes) * In(0.5)/In(RA). The calculated half-lives for the variants and the reference were used for calculation of the half-life improvement factor as the ratio between half-life of the variant and the half-life of the reference. The half-life improvement factor for the reference is by definition 1.0, and variants with half-life improvement factors larger than the reference have improved stability under the test conditions.

Single mutation variants showing improved stability in supernatant screening were used for construction and screening of combination variants comprising two or more individual mutations.

The transformations of these improved single mutation hits were streaked into single colonies, sequenced, grown overnight in TB-Gly broth with 3 µg/ml chloramphenicol. After growth in shakeflasks in 100 ml PS-1 growth medium with trace elements (see Example 1) for 4 days at 30°C the enzyme variants were purified and their stability was tested in two different liquid detergents as described below.

Single mutations resulting in improved stability were combined in combination variants containing two or more mutations compared to SEQ ID NO: 1. These were streaked to single colonies directly after transformation and then screened as described above and sequenced. Improved combination variants were subsequently grown, purified and tested for storage stability in two different liquid detergents as described below.

For variants that were subsequently tested in purified form, stability data is shown in Tables 2 and 3 in Example 3. Table 1A shows stability data for a few additional variants tested as supernatants in concentrated liquid detergent (Tide Pods^{®} 3-in-1) stored at 55°C.

Other DNase variants were tested in a similar manner as described above, although with incubation in 10% Ariel Essential detergent at a temperature of 58°C and together with 1% of a protease (SEQ ID NO: 28). The results of this test are shown in Table 1B below.

**Table 1A: Half-life Improvement Factor of DNase variants tested as supernatants in concentrated liquid detergent (Tide Pods^{®} 3-in-1)**

| **Substitutions compared to SEQ ID NO: 1** | **Half-life improvement factor** |
|---|---|
| K21L, N61 D,T65I,K107Q,T127S | 2.87 |
| Q14W,N61D,T65I,R109T,G149N,S164D,L181W | 2.18 |
| K21L, N61 D,T65I,K107Q | 2.08 |
| Q14W,N61D,T65I,R109T,G149N | 1.87 |
| Q14W,T65V,R109T,G149N,W154I,L181W | 1.65 |
| Q14W,T65V,R109T,G149N,W154I,S164D | 1.62 |
| Q14W,N61D,T65V,R109T,G149N,L181W | 1.59 |
| Q14W,T65I,R109T,D116W,G149N,S164D,L181W | 1.44 |

**Table 1B: Half-life improvement factor of DNase variants tested as supernatants in concentrated liquid detergent (Ariel Essential)**

| **Substitutions compared to SEQ ID NO: 1** | **Half-life improvement factor** |
|---|---|
| T65V,R109T,T127V,T171W | 3.38 |
| R109T,T127V,T171W | 2.63 |
| T65V,R109T,T127V | 2.02 |

### Example 3: Storage stability assay for purified DNase variants

After purification (2-step procedure using a PPA HyperCel column and an SP-Sepharose^{®} FF column), purified DNase variants were diluted with 0.01% Triton X-100 to 0.2 and 0.1 mg/ml with the concentration calculated using absorbance at 280 nm. For each enzyme variant, two wells with the high concentration (0.2 mg/ml) and two wells with the low concentration (0.1 mg/ml) were tested. 15 µl diluted DNase sample was mixed with 285 µl concentrated detergent (either 1) Tide Pods^{®} 3-in-1, green compartment with 0.3% sodium bisulfite and 1% of a liquid laundry protease, Progress^{®} Uno 101 L (Novozymes A/S) added, or 2) Tide Original HE heavy-duty liquid with 1% Progress^{®} Uno 101 L added), in the well of a microtiter plate ("detergent plate", Nunc U96 PP 0.5 ml) using a magnetic bar. After mixing the detergent plates were incubated at 50°C in a Biosan PST-100HL thermomixer.

After various incubation times (e.g. 0, 2, 24 and 72 hours), residual DNase activity was measured. 5 µl from the detergent plate was mixed with 195 µl DNA substrate solution (3.3 mg DNA (Sigma D1626) in 50 mM Tris pH 7, 5 mM MgCl₂, 5 mM CaCl₂, 1.3 mM EDTA). Once a minute for 30 minutes, viscosity was measured using pressure sensing during aspiration with a Hamilton Microlab STAR. From the measured reduction in viscosity, an activity was calculated.

The decrease in activity during incubation with detergent is assumed to be exponential. Half-lives (T½) are found from linear regression of Log(Activity) versus incubation time, and half-life improvement factors (HIF) were calculated as half-life of DNase variants relative to the half-life of a reference DNase.

The half-life improvement factor of DNase variants of the invention is shown in Tables 2 and 3 below, where Table 2 shows the results for variants tested in the concentrated liquid detergent Tide Pods^{®} 3-in-1, while Table 3 shows the results for variants tested in the heavy-duty liquid detergent Tide Original HE.

**Table 2: Half-life Improvement Factor of purified DNase variants tested in concentrated liquid detergent (Tide Pods^{®} 3-in-1)**

| **Substitutions compared to SEQ ID NO: 1** | **Half-life improvement factor** |
|---|---|
| K21L,Q48D,T65I,S82R,K107Q,T127S | 7.08 |
| Q14R,K21L,Q48D,T65I,T127S | 6.95 |
| Q48D,T65I,S82R,T127S,S164D | 6.92 |
| N61D,T65I,K107Q,T127S,S164D | 6.64 |
| Q48D,T65I,S82R,K107Q,T127S | 6.54 |
| Q14R,N61D,T65I,S82R,K107Q | 6.18 |
| N61D,S68L,G149N | 6.15 |
| Q14R,N61D,T65I,S82R,T127S,S164D | 6.11 |
| K21L,Q48D,T65I,S82R,K107Q | 5.88 |
| Q14R,T65I,K107Q,T127S | 5.62 |
| N61D,T65I,S82R,T127S,S164D | 5.59 |
| K21L,N61D,T65I,S82R,K107Q,T127S | 5.49 |
| T65V,T127V,G149N | 5.43 |
| T65I,K107Q,T127S,S164D | 5.36 |
| N61D,T65I,S82R,K107Q | 5.33 |
| Q14R,K21L,N61D,T65I,S82R | 5.28 |
| Q14R,K21L,N61D,T65I,S82R,K107Q | 5.25 |
| Q14R,K21L,N61D,T65I,T127S | 5.23 |
| N61 D,T651,S82R,K107Q,T127S,S164D | 5.21 |
| Q14R,K21L,T65I,K107Q,T127S | 5.19 |
| N61D,T65I,K107Q,T127S | 5.13 |
| T65I,S82R,K107Q,S164D | 5.00 |
| K21L,N61D,T65I,S82R | 4.83 |
| K21L,N61 D,T65I,T127S | 4.81 |
| N61D,T65I,S82R,S164D | 4.79 |
| K21L,N61 D,T65I,S82R,K107Q | 4.76 |
| S68L,S106A,G149N | 4.73 |
| N61 D,T65I,T127S,S164D | 4.67 |
| Q14R,K21 L, N61 D,T65I | 4.63 |
| Q14R,K21L,T65I,T127S | 4.55 |
| T65V,G149N | 4.36 |
| T65V,R109T,T127V | 4.21 |
| Q14R,K21L,T65I,K107Q | 4.18 |
| K21L,T65I,S82R,K107Q | 4.18 |
| K21L,T65I,K107Q,T127S | 4.15 |
| N61D,S68L,S102Y,G149N,S164D,L 181T | 3.92 |
| N61 D,S68L,S106A,G149N,S164D | 3.88 |
| T65V,R109T,G149N | 3.75 |
| T65V,T127V,T171W | 3.68 |
| T65V,T127V,L181S | 3.55 |
| T65I,S164D,L181W | 3.46 |
| N61 D,S66Y,S102Y,S164D | 3.09 |
| N61 D,S66Y,S164D | 3.05 |
| N61 D,T65V,S164D | 2.98 |
| Q14W,N61D,T65I | 2.23 |
| Q14W,N61D,T65I,S164D | 2.10 |
| Q14W,N61D,T65I,S164D,L181W | 2.06 |
| T65I,D116W,S164D,L181W | 1.88 |
| T65I,D116W,S164D | 1.78 |
| Q14W,T65I,S164D | 1.65 |
| R109T,G149N | 1.57 |
| G149N,T171W | 1.55 |
| G149N | 1.53 |
| S164D | 1.10 |

**Table 3: Half-life Improvement Factor of purified DNase variants tested in heavy-duty liquid detergent (Tide Original HE)**

| **Substitutions compared to SEQ ID NO: 1** | **Half-life improvement factor** |
|---|---|
| P25S,L33K, D56I,T65V,Y77T,T127V, L181 S | 3.57 |
| Q48D,T65I,S82R,K107Q,T127S | 3.32 |
| N61 D,T65I,S82R,K107Q,L181D | 3.30 |
| P25S,L33K,T65V,Y77T,T127V, L181 S | 3.05 |
| P25S,L33K,D56I,T65V,Y77T,R109Q,T127V,L181S | 3.00 |
| T65V,G149N,L181E | 2.93 |
| T65V,T127V,G149N,Y182D | 2.59 |
| T65V,T127V,L181S | 2.54 |
| Q48D,N61D,T651,S82R,K107Q | 2.53 |
| P25S,L33K,D56I,T65V,Y77T,D116S,T127V,L181S | 2.49 |
| D56L,T65V,T127V | 2.44 |
| Q48D,T65I,S82R,T127S,S164D | 2.43 |
| N61D,S68L,G149N | 2.38 |
| T65V,T127V,G149N | 2.35 |
| D56L,T65V,T127V,T171W | 2.34 |
| Q48D,T65V,G149N | 2.31 |
| N61D,T65I,S82R,K107Q | 2.15 |
| T65V,T127V,T171W | 2.12 |
| N61 D,T65I,S82R,T127S,S164D,Y182D | 2.04 |
| N61 D,T65I,S82R,T127S,S164D,L181D | 2.01 |
| S68L,S106A,G149N | 1.94 |
| Q48D,N61D,T651,K107Q,T127S,S164D | 1.94 |
| T65V,G149N,T171W | 1.93 |
| Q48D,T65I,K107Q,T127S,S164D | 1.83 |
| N61 D,T65I,S82R,T127S,S164D,Y182N | 1.79 |
| T65I,K107Q,T127S,S164D,Y182D | 1.76 |
| T65V,Y77T,T127V | 1.71 |
| T65V,G149N | 1.68 |
| N61 D,T65I,K107Q,T127S,S164D,L181E | 1.68 |
| N61 D,T65I,S82R,T127S,S164D | 1.65 |
| N61 D,S68L,S106A,G149N,S164D | 1.61 |
| N61 D,S66Y,S102Y,S164D | 1.60 |
| N61D,T65I,K107Q,T127S,S164D,L181D | 1.59 |
| T65I,K107Q,T127S,S164D,L181T | 1.53 |
| T65I,K107Q,T127S,S164D,L181 E | 1.48 |
| N61 D,T65I,K107Q,T127S,S164D,Y182D | 1.46 |
| T65I,K107Q,T127S,S164D,Y182N | 1.45 |
| N61D,T65I,K107Q,T127S,S164D,L181Q | 1.43 |
| N61 D,T65V,S164D,Y182N | 1.43 |
| N61 D,T65I,K107Q,T127S,S164D,L 181T | 1.42 |
| N61D,T65I,S82R,S164D | 1.41 |
| T65!,K107Q,T127S,S164D,L181 D | 1.41 |
| N61D,S66Y,S164D | 1.40 |
| N61 D,T65I,K107Q,T127S,S164D | 1.39 |
| T65I,S82R,K107Q,S164D | 1.39 |
| T65V,R109T,T127V,G149N | 1.38 |
| G149N | 1.36 |
| N61 D,T65I,T127S,S164D | 1.34 |
| T65I,K107Q,T127S,S164D,L181Q | 1.34 |
| T65V,R109T,T127V | 1.31 |
| N61 D,T65V,T127S,S164D | 1.30 |
| Q48D,N61D,T65V,S164D | 1.28 |
| T65V,R109T,G149N | 1.24 |
| T65I,K107Q,T127S,S164D | 1.20 |
| G149N,T171W | 1.19 |
| T65V,R109T,T171W | 1.18 |
| T65V,R109T,G149N,T171W | 1.16 |
| N61D,T65V,S164D | 1.15 |
| T65I,S164D,L181W | 1.11 |
| P25S, D56I,T65V,Y77T,T127V, L181 S | 1.10 |

## Claims

1. A DNase variant which compared to the polypeptide of SEQ ID NO: 1 comprises two or more substitutions selected from the group consisting of N61D, T65I, T65V, S82R, K107Q, T127S, T127V, G149N, S164D and L181S, wherein the variant has at least 60% sequence identity to SEQ ID NO: 1 and has DNase activity.

2. The DNase variant of claim 1, comprising two, three, four, five or more of said substitutions.

3. The DNase variant of claim 1 or 2, further comprising at least one substitution selected from the group consisting of Q14R, Q14W, K21L, P25S, L33K, Q48D, D56I, D56L, S66Y, S68L, Y77T, S102Y, S106A, R109Q, R109T, D116S, D116W, T171W, L181T and L181W.

4. The DNase variant of any of the preceding claims, comprising a set of substitutions selected from the group consisting of:
a) G149N together with at least one of the substitutions N61D, T65I, T65V, S82R, K107Q, T127S, T127V, S164D and L181S;
b) T65I or T65V together with at least two of the substitutions N61D, S82R, K107Q, T127S, T127V, G149N, S164D and L181S;
c) N61D together with at least two of the substitutions T65I/V, S82R, K107Q, T127S/V, G149N, S164D and L181S, preferably at least two of the substitutions T65I/V, S82R, K107Q, T127S and S164D;
d) S82R together with at least two of the substitutions N61D, T65I, T65V, K107Q, T127S, T127V, G149N, S164D and L181S;
e) K107Q together with at least two of the substitutions N61D, T65I, T65V, S82R, T127S, T127V, G149N, S164D and L181S;
f) T127S together with at least two of the substitutions N61D, T65I, T65V, S82R, K107Q, G149N, S164D and L181S; and
g) S164D together with at least one of the substitutions N61D, T65I, T65V, S82R, K107Q, T127S, T127V, G149N and L181S.

5. The DNase variant of any of the preceding claims, comprising a set of substitutions selected from the group consisting of:
• K21L+Q48D+T65I+S82R+K107Q+T127S;
• Q14R+K21L+Q48D+T65I+T127S;
• Q48D+T65I+S82R+T127S+S164D;
• N61D+T65I+K107Q+T127S+S164D;
• Q48D+T65I+S82R+K107Q+T127S;
• Q14R+N61D+T65I+S82R+K107Q;
• N61D+S68L+G149N;
• Q14R+N61D+T65I+S82R+T127S+S164D;
• K21L+Q48D+T65I+S82R+K107Q;
• Q14R+T65I+K107Q+T127S;
• N61D+T65I+S82R+T127S+S164D;
• K21L+N61D+T65I+S82R+K107Q+T127S;
• T65V+T127V+G149N;
• T65I+K107Q+T127S+S164D;
• N61D+T65I+S82R+K107Q;
• Q14R+K21L+N61D+T65I+S82R;
• Q14R+K21L+N61D+T65I+S82R+K107Q;
• Q14R+K21L+N61D+T65I+T127S;
• N61D+T65I+S82R+K107Q+T127S+S164D;
• Q14R+K21L+T65I+K107Q+T127S;
• N61D+T65I+K107Q+T127S;
• T65I+S82R+K107Q+S164D;
• K21L+N61D+T65I+S82R;
• K21L+N61D+T65I+T127S;
• N61D+T65I+S82R+S164D;
• K21L+N61D+T65I+S82R+K107Q;
• S68L+S106A+G149N;
• N61D+T65I+T127S+S164D;
• Q14R+K21L+N61D+T65I;
• Q14R+K21L+T65I+T127S;
• T65V+G149N;
• T65V+R109T+T127V;
• Q14R+K21L+T65I+K107Q;
• K21L+T65I+S82R+K107Q;
• K21L+T65I+K107Q+T127S;
• N61D+S68L+S102Y+G149N+S164D+L181T;
• N61D+S68L+S106A+G149N+S164D;
• T65V+R109T+G149N;
• T65V+T127V+T171W;
• T65V+T127V+L181S;
• T65I+S164D+L181W;
• N61D+S66Y+S102Y+S164D;
• N61D+S66Y+S164D;
• N61D+T65V+S164D;
• Q14W+N61D+T65I;
• Q14W+N61D+T65I+S164D;
• Q14W+N61D+T65I+S164D+L181W;
• T65I+D116W+S164D+L181W;
• T65I+D116W+S164D;
• Q14W+T65I+S164D;
• R109T+G149N;
• G149N+T171W;
• G149N;
• S164D;
• P25S+L33K+D56I+T65V+Y77T+T127V+L181S;
• P25S+L33K+T65V+Y77T+T127V+L181S;
• P25S+L33K+D56I+T65V+Y77T+R109Q+T127V+L181S;
• P25S+L33K+D56I+T65V+Y77T+D116S+T127V+L181S;
• D56L+T65V+T127V;
• D56L+T65V+T127V+T171W;
• T65V+G149N+T171W;
• Q48D+T65I+K107Q+T127S+S164D;
• T65V+Y77T+T127V;
• T65V+R109T+T127V+G149N;
• T65V+R109T+T171W;
• T65V+R109T+G149N+T171W;
• P25S+D56I+T65V+Y77T+T127V+L181S;
• Q14R+K21L+T65I+K107Q+T127S;
• N61D+S68L+S102Y;
• S66Y+T127V+L181S;
• N61D+T65I+S82R+K107Q+L181D;
• T65V+G149N+L181E;
• T65V+T127V+G149N+Y182D;
• Q48D+N61D+T65I+S82R+K107Q;
• Q48D+T65V+G149N;
• N61D+T65I+S82R+T127S+S164D+Y182D;
• N61D+T65I+S82R+T127S+S164D+L181D;
• Q48D+N61D+T65I+K107Q+T127S+S164D;
• N61D+T65I+S82R+T127S+S164D+Y182N;
• T65I+K107Q+T127S+S164D+Y182D;
• N61D+T65I+K107Q+T127S+S164D+L181E;
• N61D+T65I+K107Q+T127S+S164D+L181D;
• T65I+K107Q+T127S+S164D+L181T;
• T65I+K107Q+T127S+S164D+L181E;
• N61D+T65I+K107Q+T127S+S164D+Y182D;
• T65I+K107Q+T127S+S164D+Y182N;
• N61D+T65I+K107Q+T127S+S164D+L181Q;
• N61D+T65V+S164D+Y182N;
• N61D+T65I+K107Q+T127S+S164D+L181T;
• T65I+K107Q+T127S+S164D+L181D;
• T65I+K107Q+T127S+S164D+L181Q;
• N61D+T65V+T127S+S164D;
• Q48D+N61D+T65V+S164D;
• K21L+N61D+T651+K107Q+T127S;
• Q14W+N61D+T65I+R109T+G149N+S164D+L181W;
• K21L+N61D+T65I+K107Q;
• Q14W+N61D+T65I+R109T+G149N;
• Q14W+T65V+R109T+G149N+W1541+L181W;
• Q14W+T65V+R109T+G149N+W154I+S164D;
• Q14W+N61D+T65V+R109T+G149N+L181W;
• Q14W+T65I+R109T+D116W+G149N+S164D+L181W;
• T65V+R109T+T127V+T171W; and
• R109T+T127V+T171W..

6. The DNase variant of claim 5, comprising a set of substitutions selected from the group consisting of:
• T65V+T127V+L181S;
• N61D+T65I+S82R+K107Q;
• T65V+G149N;
• N61D+T65I+K107Q+T127S+S164D;
• N61D+T65I+T127S+S164D;
• N61D+T65V+S164D;
• T65V+T127V+G149N;
• N61D+T65I+S82R+T127S+S164D; and
• T65I+K107Q+T127S+S164D.

7. The DNase variant of claim 5 or 6, wherein the variant comprises or consists of the polypeptide of SEQ ID NO: 1 with one of said sets of substitutions.

8. The DNase variant of any of the preceding claims, wherein the variant has at least 70%, at least 80%, at least 85%, at least 90%, at least 91%, at least 92%, at least 93%, at least 94%, at least 95%, at least 96%, at least 97%, at least 97% or at least 98% sequence identity to SEQ ID NO: 1.

9. The DNase variant of any of the preceding claims, wherein the variant comprises at least 5, such as at least 10, such as at least 15, of the indicated amino acid residues in the following positions: I in position 1, Y in position 13, P in position 22, P in position 25, L in position 27, P in position 39, G in position 42, W in position 57, V in position 59, L in position 76, Y in position 77, R in position 109, D in position 116, P in position 144, H in position 147, L in position 167, D in position 175 and L in position 181.

10. The DNase variant of claim 9, wherein the variant comprises 15, 16, 17 or 18 of the indicated amino acid residues in the following positions: I in position 1, Y in position 13, P in position 22, P in position 25, L in position 27, P in position 39, G in position 42, W in position 57, V in position 59, L in position 76, Y in position 77, R in position 109, D in position 116, P in position 144, H in position 147, L in position 167, D in position 175 and L in position 181.

11. The DNase variant of any of the preceding claims, wherein the variant comprises one or more substitutions selected from the group consisting of 11T, Y13S, P22T, P25S, L27S, P39S, G42S, W57S, V59S, L76V, Y77T, R109Q, D116S, P144S, H147A, L167S and D175G.

12. A method for obtaining a DNase variant, comprising:
a) introducing into a parent DNase having at least 60% sequence identity to SEQ ID NO: 1 two or more substitutions selected from the group consisting of N61D, T65I, T65V, S82R, K107Q, T127S, T127V, G149N, S164D and L181S (numbering based on SEQ ID NO: 1), and
b) recovering the variant,
wherein the variant has DNase activity.

13. A nucleic acid construct comprising a polynucleotide encoding a DNase polypeptide as defined in any of claims 1-11, wherein the polynucleotide is operably linked to one or more control sequences that direct expression of the polypeptide.

14. A recombinant host cell comprising the nucleic acid construct of claim 13.

15. A method of producing a DNase polypeptide, comprising:
a) cultivating the host cell of claim 14 under conditions suitable for expression of the polypeptide; and, optionally,
b) recovering the polypeptide.


SEQUENCE LISTING

<110>  Novozymes A/S

<120>  POLYPEPTIDE VARIANTS

<130>  15221-EP-EPA

<160>  28

<170>  PatentIn version 3.5

<210>  1
<211>  182
<212>  PRT
<213>  Artificial Sequence

<220>
<223>  Variant

<400>  1

Ile Pro Pro Gly Thr Pro Ser Lys Ser Ala Ala Gln Tyr Gln Leu Asn
1               5                   10                  15

Ala Leu Thr Val Lys Pro Glu Gly Pro Met Leu Gly Tyr Ser Arg Asp
            20                  25                  30

Leu Phe Pro His Trp Ile Pro Gln Gly Gly Gly Cys Asp Thr Arg Gln
        35                  40                  45

Val Val Leu Lys Arg Asp Ala Asp Trp Tyr Val Gly Asn Cys Pro Val
    50                  55                  60

Thr Ser Gly Ser Trp Tyr Ser Tyr Tyr Asp Gly Leu Tyr Phe Thr Asn
65                  70                  75                  80

Pro Ser Asp Leu Asp Ile Asp His Ile Val Pro Leu Ala Glu Ala Trp
                85                  90                  95

Arg Ser Gly Ala Ser Ser Trp Thr Thr Ser Lys Arg Arg Asp Phe Ala
            100                 105                 110

Asn Asp Leu Asp Gly Pro Gln Leu Ile Ala Val Ser Ala Ser Thr Asn
        115                 120                 125

Arg Ser Lys Gly Asp Gln Asp Pro Ser Thr Trp Gln Pro Pro Arg Pro
    130                 135                 140

Gly Ala His Cys Gly Tyr Ser Lys Trp Trp Ile Ser Thr Lys Tyr Lys
145                 150                 155                 160

Trp Gly Leu Ser Leu Gln Leu Ser Glu Lys Thr Ala Leu Gln Asp Met

                165                 170                 175

Leu Asn Ser Cys Leu Tyr
            180

<210> 2
<211> 182
<212> PRT
<213> Bacillus horikoshii

<400> 2

Leu Pro Pro Gly Thr Pro Thr Lys Ser Glu Ala Gln Asn Gln Leu Asn
1               5                   10                  15

Ser Leu Thr Val Lys Ser Glu Gly Ser Met Thr Gly Tyr Ser Arg Asp
            20                  25                  30

Leu Phe Pro His Trp Ser Gly Gln Gly Asn Gly Cys Asp Thr Arg Gln
        35                  40                  45

Ile Val Leu Gln Arg Asp Ala Asp Tyr Tyr Thr Gly Thr Cys Pro Thr
    50                  55                  60

Thr Ser Gly Lys Trp Tyr Ser Tyr Phe Asp Gly Val Ile Val Tyr Ser
65                  70                  75                  80

Pro Ser Glu Ile Asp Ile Asp His Ile Val Pro Leu Ala Glu Ala Trp
                85                  90                  95

Arg Ser Gly Ala Ser Ser Trp Thr Thr Glu Gln Arg Arg Ala Phe Ala
            100                 105                 110

Asn Asp Leu Asn Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Ala
    130                 135                 140

Gly Ala Arg Cys Ala Tyr Ala Lys Trp Trp Ile Asn Thr Lys His Arg
145                 150                 155                 160

Trp Asn Leu His Leu Gln Ser Ser Glu Lys Ser Ser Leu Gln Thr Met
                165                 170                 175

Leu Asn Gly Cys Ala Tyr
            180

<210> 3

<211>  182
<212>  PRT
<213>  Bacillus sp-62520

<400>  3

Leu Pro Pro Gly Thr Pro Ser Lys Ser Glu Ala Gln Ser Gln Leu Asn
1               5                   10                  15

Ala Leu Thr Val Lys Pro Glu Asp Pro Met Thr Gly Tyr Ser Arg Asp
            20                  25                  30

His Phe Pro His Trp Ile Ser Gln Gly Asn Gly Cys Asn Thr Arg Gln
        35                  40                  45

Ile Val Leu Gln Arg Asp Ala Asp Tyr Tyr Ser Gly Ala Cys Pro Val
    50                  55                  60

Thr Thr Gly Lys Trp Tyr Ser Tyr Phe Asp Gly Val Ile Val Tyr Ser
65                  70                  75                  80

Pro Ser Glu Ile Asp Ile Asp His Ile Val Pro Leu Ala Glu Ala Trp
                85                  90                  95

Arg Ser Gly Ala Ser Ser Trp Thr Thr Glu Lys Arg Arg Ser Phe Ala
            100                 105                 110

Asn Asp Leu Asn Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Ala
    130                 135                 140

Gly Ala Arg Cys Ala Tyr Ala Lys Trp Trp Ile Asn Thr Lys His Arg
145                 150                 155                 160

Trp Gly Leu His Leu Gln Ser Ser Glu Lys Ser Ser Leu Gln Ser Met
                165                 170                 175

Leu Asn Gly Cys Ala Tyr
            180


<210>  4
<211>  182
<212>  PRT
<213>  Bacillus horikoshii

<400>  4

Leu Pro Pro Gly Thr Pro Ser Lys Ser Glu Ala Gln Ser Gln Leu Asn
1               5                   10                  15

Ser Leu Thr Val Lys Ser Glu Asp Pro Met Thr Gly Tyr Ser Arg Asp
            20                  25                  30

His Phe Pro His Trp Ser Gly Gln Gly Asn Gly Cys Asp Thr Arg Gln
        35                  40                  45

Ile Val Leu Gln Arg Asp Ala Asp Tyr Tyr Ser Gly Asn Cys Pro Val
    50                  55                  60

Thr Ser Gly Lys Trp Tyr Ser Tyr Phe Asp Gly Val Ile Val Tyr Ser
65                  70                  75                  80

Pro Ser Glu Ile Asp Ile Asp His Val Val Pro Leu Ala Glu Ala Trp
                85                  90                  95

Arg Ser Gly Ala Ser Ser Trp Thr Thr Glu Gln Arg Arg Ser Phe Ala
            100                 105                 110

Asn Asp Leu Asn Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Ala
    130                 135                 140

Gly Ala Arg Cys Ala Tyr Ala Lys Trp Trp Ile Asn Thr Lys His Arg
145                 150                 155                 160

Trp Asn Leu His Leu Gln Ser Ser Glu Lys Ser Ala Leu Gln Thr Met
                165                 170                 175

Leu Asn Gly Cys Val Tyr
            180

<210>  5
<211>  182
<212>  PRT
<213>  Bacillus horikoshii

<400>  5

Leu Pro Pro Gly Thr Pro Ser Lys Ser Glu Ala Gln Ser Gln Leu Asn
1               5                   10                  15

Ser Leu Thr Val Lys Thr Glu Asp Pro Met Thr Gly Tyr Ser Arg Asp
            20                  25                  30

Leu Phe Pro His Trp Ser Gly Gln Gly Ser Gly Cys Asp Thr Arg Gln
        35                  40                  45

Ile Val Leu Gln Arg Asp Ala Asp Tyr Phe Thr Gly Thr Cys Pro Thr
    50                  55                  60

Thr Ser Gly Lys Trp Tyr Ser Tyr Phe Asp Gly Val Ile Val Tyr Ser
65                  70                  75                  80

Pro Ser Glu Ile Asp Val Asp His Ile Val Pro Leu Ala Glu Ala Trp
                85                  90                  95

Arg Ser Gly Ala Ser Ser Trp Thr Thr Glu Gln Arg Arg Ala Phe Ala
            100                 105                 110

Asn Asp Leu Thr Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Ala
    130                 135                 140

Gly Ala Arg Cys Ala Tyr Ala Lys Trp Trp Ile Asn Thr Lys His Arg
145                 150                 155                 160

Trp Asn Leu His Leu Gln Ser Ser Glu Lys Ser Ser Leu Gln Thr Met
                165                 170                 175

Leu Asn Gly Cys Ala Tyr
            180

<210>  6
<211>  182
<212>  PRT
<213>  Bacillus sp-16840

<400>  6

Leu Pro Pro Gly Thr Pro Ser Lys Ser Glu Ala Gln Ser Gln Leu Asn
1               5                   10                  15

Ala Leu Thr Val Lys Ala Glu Asp Pro Met Thr Gly Tyr Ser Arg Asn
            20                  25                  30

Leu Phe Pro His Trp Asn Ser Gln Gly Asn Gly Cys Asn Thr Arg Gln
        35                  40                  45

Leu Val Leu Gln Arg Asp Ala Asp Tyr Tyr Ser Gly Asn Cys Pro Val
    50                  55                  60

Thr Ser Gly Arg Trp Tyr Ser Tyr Phe Asp Gly Val Val Val Thr Ser
65                  70                  75                  80

Pro Ser Glu Ile Asp Ile Asp His Ile Val Pro Leu Ala Glu Ala Trp
                85                  90                  95

Arg Ser Gly Ala Ser Ser Trp Thr Thr Glu Lys Arg Lys Glu Phe Ala
            100                 105                 110

Asn Asp Leu Asn Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Ala
    130                 135                 140

Ala Ala Arg Cys Gly Tyr Ala Lys Trp Trp Ile Asn Thr Lys Tyr Arg
145                 150                 155                 160

Trp Asp Leu Ser Leu Gln Ser Ser Glu Lys Ser Ser Leu Gln Thr Met
                165                 170                 175

Leu Asn Thr Cys Ser Tyr
            180

<210> 7
<211> 182
<212> PRT
<213> Bacillus sp-16840

<400> 7

Leu Pro Pro Gly Thr Pro Ser Lys Ser Gln Ala Gln Ser Gln Leu Asn
1               5                   10                  15

Ala Leu Thr Val Lys Ala Glu Asp Pro Met Thr Gly Tyr Ser Arg Asn
            20                  25                  30

Leu Phe Pro His Trp Ser Ser Gln Gly Asn Gly Cys Asn Thr Arg Gln
        35                  40                  45

Leu Val Leu Gln Arg Asp Ala Asp Tyr Tyr Ser Gly Asn Cys Pro Val
    50                  55                  60

Thr Ser Gly Arg Trp Tyr Ser Tyr Phe Asp Gly Val Val Val Thr Ser
65                  70                  75                  80

Pro Ser Glu Ile Asp Ile Asp His Ile Val Pro Leu Ala Glu Ala Trp
                85                  90                  95

Arg Ser Gly Ala Ser Ser Trp Thr Thr Glu Lys Arg Arg Glu Phe Ala
            100                 105                 110

Asn Asp Leu Asn Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Val
    130                 135                 140

Ala Ala Arg Cys Gly Tyr Ala Lys Trp Trp Ile Asn Thr Lys Tyr Arg
145                 150                 155                 160

Trp Asp Leu Ser Leu Gln Ser Ser Glu Lys Ser Ser Leu Gln Thr Met
                165                 170                 175

Leu Asn Thr Cys Ser Tyr
            180

<210>  8
<211>  182
<212>  PRT
<213>  Bacillus sp-62668

<400>  8

Leu Pro Pro Gly Thr Pro Ser Lys Ser Glu Ala Gln Ser Gln Leu Thr
1               5                   10                  15

Ser Leu Thr Val Lys Pro Glu Asp Pro Met Thr Gly Tyr Ser Arg Asp
            20                  25                  30

His Phe Pro His Trp Ile Ser Gln Gly Asn Gly Cys Asn Thr Arg Gln
        35                  40                  45

Ile Val Leu Gln Arg Asp Ala Asp Tyr Tyr Ser Gly Asn Cys Pro Val
    50                  55                  60

Thr Thr Gly Lys Trp Tyr Ser Tyr Phe Asp Gly Val Ile Val Tyr Ser
65                  70                  75                  80

Pro Ser Glu Ile Asp Ile Asp His Ile Val Pro Leu Ala Glu Ala Trp
                85                  90                  95

Arg Ser Gly Ala Ser Ser Trp Thr Ala Glu Gln Arg Arg Asn Phe Ala
            100                 105                 110

Asn Asp Leu Asn Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Thr
    130                 135                 140

Gly Ala Arg Cys Ala Tyr Ala Lys Trp Trp Ile Asn Thr Lys Tyr Arg
145                 150                 155                 160

Trp Gly Leu His Leu Gln Ser Ser Glu Lys Ser Ser Leu Gln Ser Met
                165                 170                 175

Leu Asn Gly Cys Ala Tyr
            180

<210>  9
<211>  185
<212>  PRT
<213>  Bacillus sp-13395

<400>  9

Ala Ser Ala Phe Pro Pro Gly Thr Pro Ser Lys Ser Thr Ala Gln Ser
1               5                   10                  15

Gln Leu Asn Ser Leu Pro Val Lys Ser Glu Gly Ser Met Asn Gly Tyr
            20                  25                  30

Ser Arg Asp Lys Phe Pro His Trp Ile Ser Gln Gly Asp Gly Cys Asp
        35                  40                  45

Thr Arg Gln Leu Val Leu Lys Arg Asp Gly Asp Tyr Tyr Ser Gly Ser
    50                  55                  60

Cys Pro Val Thr Ser Gly Lys Trp Tyr Ser Tyr Tyr Asp Gly Ile Thr
65                  70                  75                  80

Val Tyr Ser Pro Ser Glu Ile Asp Ile Asp His Ile Val Pro Leu Ala
                85                  90                  95

Glu Ala Trp Arg Ser Gly Ala Ser Gly Trp Thr Thr Glu Lys Arg Gln
            100                 105                 110

Ser Phe Ala Asn Asp Leu Asn Gly Pro Gln Leu Ile Ala Val Thr Ala
        115                 120                 125

Ser Val Asn Arg Ser Lys Gly Asp Gln Asp Pro Ser Thr Trp Gln Pro
    130                 135                 140

Pro Arg Ser Gly Ser His Cys Ala Tyr Ala Lys Met Trp Val Asn Thr
145                 150                 155                 160

Lys Tyr Arg Trp Gly Leu His Leu Gln Ser Ala Glu Lys Ser Ala Leu
                165                 170                 175

Gln Ser Met Leu Asn Ala Cys Ser Tyr
            180                 185

<210> 10
<211> 185
<212> PRT
<213> Bacillus horneckiae

<400> 10

Ala Ser Ala Phe Pro Pro Gly Thr Pro Ser Lys Ser Thr Ala Gln Ser
1               5                   10                  15

Gln Leu Asn Ser Leu Thr Val Lys Ser Glu Gly Ser Met Thr Gly Tyr
            20                  25                  30

Ser Arg Asp Lys Phe Pro His Trp Ile Ser Gln Gly Asp Gly Cys Asp
        35                  40                  45

Thr Arg Gln Leu Val Leu Lys Arg Asp Gly Asp Tyr Tyr Ser Gly Asn
    50                  55                  60

Cys Pro Val Thr Ser Gly Lys Trp Tyr Ser Tyr Tyr Asp Gly Ile Thr
65                  70                  75                  80

Val Tyr Ser Pro Ser Glu Ile Asp Ile Asp His Ile Val Pro Leu Ala
                85                  90                  95

Glu Ala Trp Arg Ser Gly Ala Ser Gly Trp Thr Thr Glu Lys Arg Gln
            100                 105                 110

Ser Phe Ala Asn Asp Leu Asn Gly Pro Gln Leu Ile Ala Val Thr Ala
        115                 120                 125

Ser Val Asn Arg Ser Lys Gly Asp Gln Asp Pro Ser Thr Trp Gln Pro
    130                 135                 140

Pro Arg Ser Gly Ser His Cys Ala Tyr Ala Lys Met Trp Val Asn Thr
145                 150                 155                 160

Lys Tyr Arg Trp Gly Leu His Val Gln Ser Ala Glu Lys Ser Ala Leu
                165                 170                 175

Gln Ser Met Leu Asn Ala Cys Ser Tyr
            180                 185

<210> 11
<211> 182

<212>  PRT
<213>  Bacillus sp-11238

<400>  11

Phe Pro Pro Glu Ile Pro Ser Lys Ser Thr Ala Gln Ser Gln Leu Asn
1               5                   10                  15

Ser Leu Thr Val Lys Ser Glu Asp Ala Met Thr Gly Tyr Ser Arg Asp
            20                  25                  30

Lys Phe Pro His Trp Ile Ser Gln Gly Asp Gly Cys Asp Thr Arg Gln
        35                  40                  45

Met Val Leu Lys Arg Asp Ala Asp Tyr Tyr Ser Gly Ser Cys Pro Val
    50                  55                  60

Thr Ser Gly Lys Trp Tyr Ser Tyr Tyr Asp Gly Ile Thr Val Tyr Ser
65                  70                  75                  80

Pro Ser Glu Ile Asp Ile Asp His Ile Val Pro Leu Ala Glu Ala Trp
                85                  90                  95

Arg Ser Gly Ala Ser Ser Trp Thr Thr Glu Lys Arg Arg Asn Phe Ala
            100                 105                 110

Asn Asp Leu Asn Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Ser
    130                 135                 140

Gly Ala Arg Cys Ala Tyr Ala Lys Met Trp Val Asn Thr Lys Tyr Arg
145                 150                 155                 160

Trp Gly Leu His Leu Gln Ser Ala Glu Lys Ser Gly Leu Glu Ser Met
                165                 170                 175

Leu Asn Thr Cys Ser Tyr
            180


<210>  12
<211>  182
<212>  PRT
<213>  Bacillus SP-62451

<400>  12

Leu Pro Pro Asp Leu Pro Ser Lys Ser Thr Thr Gln Ala Gln Leu Asn
1               5                   10                  15

Ser Leu Asn Val Lys Asn Glu Glu Ser Met Ser Gly Tyr Ser Arg Glu
            20                  25                  30

Lys Phe Pro His Trp Ile Ser Gln Gly Asp Gly Cys Asp Thr Arg Gln
        35                  40                  45

Val Ile Leu Lys Arg Asp Ala Asp Asn Tyr Ser Gly Asn Cys Pro Val
    50                  55                  60

Thr Ser Gly Lys Trp Tyr Ser Tyr Tyr Asp Gly Ile Thr Phe Asn Asp
65                  70                  75                  80

Pro Ser Gln Leu Asp Ile Asp His Val Val Pro Leu Ala Glu Ala Trp
                85                  90                  95

Arg Ser Gly Ala Ser Ser Trp Ser Thr Ala Lys Arg Glu Asp Phe Ala
            100                 105                 110

Asn Asp Leu Asn Gly Pro Gln Leu Ile Ala Val Ser Ala Ser Ser Asn
        115                 120                 125

Arg Ser Lys Gly Asp Gln Asp Pro Ser Thr Trp Gln Pro Pro Arg Ala
    130                 135                 140

Gly Ala Asn Cys Ala Tyr Ala Lys Met Trp Ile Asn Thr Lys Tyr Asn
145                 150                 155                 160

Trp Gly Leu His Leu Gln Ser Ser Glu Lys Thr Ala Leu Gln Gly Met
                165                 170                 175

Leu Asn Ser Cys Ser Tyr
            180

<210>  13
<211>  182
<212>  PRT
<213>  Bacillus sp-18318

<400>  13

Phe Pro Pro Gly Thr Pro Ser Lys Ser Thr Ala Gln Ser Gln Leu Asn
1               5                   10                  15

Ser Leu Thr Val Lys Ser Glu Gly Ser Met Thr Gly Tyr Ser Arg Asp
            20                  25                  30

Lys Phe Pro His Trp Ile Gly Gln Gly Ser Gly Cys Asp Thr Arg Gln
        35                  40                  45

Leu Val Leu Gln Arg Asp Ala Asp Tyr Tyr Ser Gly Ser Cys Pro Val
    50                  55                  60

Thr Ser Gly Lys Trp Tyr Ser Tyr Tyr Asp Gly Val Thr Phe Tyr Asp
65                  70                  75                  80

Pro Ser Asp Leu Asp Ile Asp His Val Val Pro Leu Ala Glu Ala Trp
                85                  90                  95

Arg Ser Gly Ala Ser Ser Trp Ser Thr Gln Lys Arg Lys Asp Phe Ala
            100                 105                 110

Asn Asp Leu Ser Gly Pro Gln Leu Ile Ala Val Ser Ala Ser Ser Asn
        115                 120                 125

Arg Ser Lys Gly Asp Gln Asp Pro Ser Thr Trp Gln Pro Thr Arg Ser
    130                 135                 140

Gly Ala Ala Cys Gly Tyr Ser Lys Trp Trp Ile Ser Thr Lys His Lys
145                 150                 155                 160

Trp Gly Leu Ser Leu Gln Ser Ser Glu Lys Asn Ala Leu Gln Gly Met
                165                 170                 175

Leu Asn Ser Cys Val Tyr
            180

<210>  14
<211>  182
<212>  PRT
<213>  Bacillus idriensis

<400>  14

Leu Pro Pro Gly Thr Pro Ser Lys Ser Thr Ala Gln Ser Gln Leu Asn
1               5                   10                  15

Ala Leu Thr Val Gln Thr Glu Gly Ser Met Thr Gly Tyr Ser Arg Asp
            20                  25                  30

Lys Phe Pro His Trp Ile Ser Gln Gly Asn Gly Cys Asp Thr Arg Gln
        35                  40                  45

Val Val Leu Gln Arg Asp Ala Asp Tyr Tyr Ser Gly Thr Cys Pro Val
    50                  55                  60

Thr Ser Gly Lys Trp Tyr Ser Tyr Tyr Asp Gly Val Thr Leu Tyr Asn
65                  70                  75                  80

Pro Ser Asp Leu Asp Ile Asp His Val Val Ala Leu Ala Glu Ala Trp
                85                  90                  95

Arg Ser Gly Ala Ser Ser Trp Thr Thr Asp Lys Arg Glu Asp Phe Ala
            100                 105                 110

Asn Asp Leu Ser Gly Thr Gln Leu Ile Ala Val Ser Ala Ser Thr Asn
        115                 120                 125

Arg Ser Lys Gly Asp Gln Asp Pro Ser Thr Trp Gln Pro Pro Arg Ser
    130                 135                 140

Gly Ala Ala Cys Gly Tyr Ala Lys Trp Trp Ile Ser Thr Lys Tyr Lys
145                 150                 155                 160

Trp Asn Leu Asn Leu Gln Ser Ser Glu Lys Thr Ala Leu Gln Ser Met
                165                 170                 175

Leu Asn Ser Cys Ser Tyr
            180

<210>  15
<211>  182
<212>  PRT
<213>  Bacillus algicola

<400>  15

Phe Pro Pro Gly Thr Pro Ser Lys Ser Glu Ala Gln Ser Gln Leu Asn
1               5                   10                  15

Ser Leu Thr Val Gln Ser Glu Gly Ser Met Ser Gly Tyr Ser Arg Asp
            20                  25                  30

Lys Phe Pro His Trp Ile Gly Gln Gly Asn Gly Cys Asp Thr Arg Gln
        35                  40                  45

Leu Val Leu Gln Arg Asp Ala Asp Tyr Tyr Ser Gly Asp Cys Pro Val
    50                  55                  60

Thr Ser Gly Lys Trp Tyr Ser Tyr Phe Asp Gly Val Thr Val Tyr Asp
65                  70                  75                  80

Pro Ser Asp Leu Asp Ile Asp His Met Val Pro Met Ala Glu Ala Trp
                85                  90                  95

Arg Ser Gly Ala Ser Ser Trp Ser Thr Gln Lys Arg Glu Asp Phe Ala
            100                 105                 110

Asn Asp Leu Ser Gly Pro His Leu Ile Ala Val Thr Ala Ser Ser Asn
        115                 120                 125

Arg Ser Lys Gly Asp Gln Asp Pro Ser Thr Trp Lys Pro Thr Arg Tyr
    130                 135                 140

Gly Ala His Cys Gly Tyr Ala Lys Trp Trp Ile Asn Thr Lys Tyr Val
145                 150                 155                 160

Tyr Asp Leu Thr Leu Gln Ser Ser Glu Lys Thr Glu Leu Gln Ser Met
                165                 170                 175

Leu Asn Thr Cys Ser Tyr
            180

<210>  16
<211>  182
<212>  PRT
<213>  Enviromental sample J

<400>  16

Leu Pro Pro Asn Ile Pro Ser Lys Ala Asp Ala Leu Thr Lys Leu Asn
1               5                   10                  15

Ala Leu Thr Val Gln Thr Glu Gly Pro Met Thr Gly Tyr Ser Arg Asp
            20                  25                  30

Leu Phe Pro His Trp Ser Ser Gln Gly Asn Gly Cys Asn Thr Arg His
        35                  40                  45

Val Val Leu Lys Arg Asp Ala Asp Ser Val Val Asp Thr Cys Pro Val
    50                  55                  60

Thr Thr Gly Arg Trp Tyr Ser Tyr Tyr Asp Gly Leu Val Phe Thr Ser
65                  70                  75                  80

Ala Ser Asp Ile Asp Ile Asp His Val Val Pro Leu Ala Glu Ala Trp
                85                  90                  95

Arg Ser Gly Ala Ser Ser Trp Thr Ser Thr Lys Arg Gln Ser Phe Ala
            100                 105                 110

Asn Asp Leu Asn Gly Pro Gln Leu Ile Ala Val Ser Ala Thr Ser Asn
        115                 120                 125

Arg Ser Lys Gly Asp Gln Asp Pro Ser Thr Trp Gln Pro Pro Arg Ala
    130                 135                 140

Gly Ala Arg Cys Ala Tyr Ala Lys Met Trp Val Glu Thr Lys Ser Arg
145                 150                 155                 160

Trp Gly Leu Thr Leu Gln Ser Ser Glu Lys Ala Ala Leu Gln Thr Ala
                165                 170                 175

Ile Asn Ala Cys Ser Tyr
            180

<210>  17
<211>  182
<212>  PRT
<213>  Bacillus vietnamensis

<400>  17

Phe Pro Pro Gly Thr Pro Ser Lys Ser Thr Ala Gln Ser Gln Leu Asn
1               5                   10                  15

Ala Leu Thr Val Lys Ser Glu Ser Ser Met Thr Gly Tyr Ser Arg Asp
            20                  25                  30

Lys Phe Pro His Trp Ile Gly Gln Arg Asn Gly Cys Asp Thr Arg Gln
        35                  40                  45

Leu Val Leu Gln Arg Asp Ala Asp Ser Tyr Ser Gly Ser Cys Pro Val
    50                  55                  60

Thr Ser Gly Ser Trp Tyr Ser Tyr Tyr Asp Gly Val Thr Phe Thr Asp
65                  70                  75                  80

Pro Ser Asp Leu Asp Ile Asp His Val Val Pro Leu Ala Glu Ala Trp
                85                  90                  95

Arg Ser Gly Ala Ser Ser Trp Thr Thr Ala Lys Arg Glu Asp Phe Ala
            100                 105                 110

Asn Asp Leu Ser Gly Pro Gln Leu Ile Ala Val Ser Ala Ser Ser Asn
        115                 120                 125

Arg Ser Lys Gly Asp Gln Asp Pro Ser Thr Trp Gln Pro Pro Arg Ser
    130                 135                 140

Gly Ala Ala Cys Gly Tyr Ser Lys Trp Trp Ile Ser Thr Lys Tyr Lys
145                 150                 155                 160

Trp Gly Leu Ser Leu Gln Ser Ser Glu Lys Thr Ala Leu Gln Gly Met
                165                 170                 175

Leu Asn Ser Cys Ile Tyr
                180

<210>  18
<211>  182
<212>  PRT
<213>  Bacillus hwajinpoensis

<400>  18

Ile Pro Pro Gly Thr Pro Ser Lys Ser Ala Ala Gln Ser Gln Leu Asp
1               5                   10                  15

Ser Leu Ala Val Gln Ser Glu Gly Ser Met Ser Gly Tyr Ser Arg Asp
            20                  25                  30

Lys Phe Pro His Trp Ile Gly Gln Gly Asn Gly Cys Asp Thr Arg Gln
        35                  40                  45

Leu Val Leu Gln Arg Asp Ala Asp Tyr Tyr Ser Gly Asp Cys Pro Val
    50                  55                  60

Thr Ser Gly Lys Trp Tyr Ser Tyr Phe Asp Gly Val Gln Val Tyr Asp
65                  70                  75                  80

Pro Ser Tyr Leu Asp Ile Asp His Met Val Pro Leu Ala Glu Ala Trp
                85                  90                  95

Arg Ser Gly Ala Ser Ser Trp Ser Thr Gln Lys Arg Glu Asp Phe Ala
            100                 105                 110

Asn Asp Leu Asp Gly Pro His Leu Ile Ala Val Thr Ala Ser Ser Asn
        115                 120                 125

Arg Ser Lys Gly Asp Gln Asp Pro Ser Thr Trp Lys Pro Thr Arg Tyr
    130                 135                 140

Ser Ala His Cys Gly Tyr Ala Lys Trp Trp Ile Asn Thr Lys Tyr Val
145                 150                 155                 160

Tyr Asp Leu Asn Leu Gln Ser Ser Glu Lys Ser Ala Leu Gln Ser Met
                165                 170                 175

Leu Asn Thr Cys Ser Tyr
            180

<210>  19
<211>  182
<212>  PRT

<213>  Paenibacillus mucilaginosus

<400>  19

Leu Pro Pro Gly Thr Pro Ser Lys Ser Thr Ala Gln Ser Gln Leu Asn
1               5                   10                  15

Ser Leu Thr Val Lys Ser Glu Ser Thr Met Thr Gly Tyr Ser Arg Asp
            20                  25                  30

Lys Phe Pro His Trp Thr Ser Gln Gly Gly Gly Cys Asp Thr Arg Gln
        35                  40                  45

Val Val Leu Lys Arg Asp Ala Asp Tyr Tyr Ser Gly Ser Cys Pro Val
    50                  55                  60

Thr Ser Gly Lys Trp Tyr Ser Tyr Tyr Asp Gly Ile Thr Val Tyr Ser
65                  70                  75                  80

Pro Ser Glu Ile Asp Ile Asp His Ile Val Pro Leu Ala Glu Ala Trp
                85                  90                  95

Arg Ser Gly Ala Ser Ser Trp Thr Thr Glu Lys Arg Gln Asn Phe Ala
            100                 105                 110

Asn Asp Leu Gly Gly Pro Gln Leu Ile Ala Val Thr Ala Ser Ser Asn
        115                 120                 125

Arg Ala Lys Gly Asp Gln Asp Pro Ser Thr Trp Lys Pro Thr Arg Ser
    130                 135                 140

Gly Ala His Cys Ala Tyr Ala Lys Trp Trp Ile Asn Thr Lys Tyr Arg
145                 150                 155                 160

Trp Gly Leu His Leu Gln Ser Ser Glu Lys Thr Ala Leu Gln Ser Met
                165                 170                 175

Leu Asn Thr Cys Ser Tyr
            180


<210>  20
<211>  182
<212>  PRT
<213>  Bacillus indicus

<400>  20

Thr Pro Pro Gly Thr Pro Ser Lys Ser Thr Ala Gln Thr Gln Leu Asn
1               5                   10                  15

Ala Leu Thr Val Lys Thr Glu Gly Ser Met Thr Gly Tyr Ser Arg Asp
            20                  25                  30

Leu Phe Pro His Trp Ile Ser Gln Gly Ser Gly Cys Asp Thr Arg Gln
        35                  40                  45

Val Val Leu Lys Arg Asp Ala Asp Tyr Tyr Ser Gly Ser Cys Pro Val
    50                  55                  60

Thr Ser Gly Lys Trp Tyr Ser Tyr Tyr Asp Gly Val Thr Phe Tyr Asp
65                  70                  75                  80

Pro Ser Asp Leu Asp Ile Asp His Ile Val Pro Leu Ala Glu Ala Trp
                85                  90                  95

Arg Ser Gly Ala Ser Ser Trp Thr Thr Ser Lys Arg Gln Asp Phe Ala
            100                 105                 110

Asn Asp Leu Ser Gly Pro Gln Leu Ile Ala Val Ser Ala Ser Thr Asn
        115                 120                 125

Arg Ser Lys Gly Asp Gln Asp Pro Ser Thr Trp Gln Pro Pro Arg Ala
    130                 135                 140

Gly Ala Ala Cys Gly Tyr Ser Lys Trp Trp Ile Ser Thr Lys Tyr Lys
145                 150                 155                 160

Trp Gly Leu Ser Leu Gln Ser Ser Glu Lys Thr Ala Leu Gln Gly Met
                165                 170                 175

Leu Asn Ser Cys Ser Tyr
            180

<210>  21
<211>  182
<212>  PRT
<213>  Bacillus marisflavi

<400>  21

Thr Pro Pro Val Thr Pro Ser Lys Ala Thr Ser Gln Ser Gln Leu Asn
1               5                   10                  15

Gly Leu Thr Val Lys Thr Glu Gly Ala Met Thr Gly Tyr Ser Arg Asp
            20                  25                  30

Lys Phe Pro His Trp Ser Ser Gln Gly Gly Gly Cys Asp Thr Arg Gln
        35                  40                  45

Val Val Leu Lys Arg Asp Ala Asp Ser Tyr Ser Gly Asn Cys Pro Val
    50                  55                  60

Thr Ser Gly Ser Trp Tyr Ser Tyr Tyr Asp Gly Val Lys Phe Thr Asn
65                  70                  75                  80

Pro Ser Asp Leu Asp Ile Asp His Ile Val Pro Leu Ala Glu Ala Trp
                85                  90                  95

Arg Ser Gly Ala Ser Ser Trp Thr Thr Ala Gln Arg Glu Ala Phe Ala
            100                 105                 110

Asn Asp Leu Ser Gly Ser Gln Leu Ile Ala Val Ser Ala Ser Ser Asn
        115                 120                 125

Arg Ser Lys Gly Asp Gln Asp Pro Ser Thr Trp Gln Pro Pro Arg Ala
    130                 135                 140

Gly Ala Lys Cys Gly Tyr Ala Lys Trp Trp Ile Ser Thr Lys Ser Lys
145                 150                 155                 160

Trp Asn Leu Ser Leu Gln Ser Ser Glu Lys Thr Ala Leu Gln Gly Met
                165                 170                 175

Leu Asn Ser Cys Val Tyr
            180

<210>  22
<211>  184
<212>  PRT
<213>  Bacillus luciferensis

<400>  22

Ala Ser Leu Pro Pro Gly Ile Pro Ser Leu Ser Thr Ala Gln Ser Gln
1               5                   10                  15

Leu Asn Ser Leu Thr Val Lys Ser Glu Gly Ser Leu Thr Gly Tyr Ser
            20                  25                  30

Arg Asp Val Phe Pro His Trp Ile Ser Gln Gly Ser Gly Cys Asp Thr
        35                  40                  45

Arg Gln Val Val Leu Lys Arg Asp Ala Asp Tyr Tyr Ser Gly Asn Cys
    50                  55                  60

Pro Val Thr Ser Gly Lys Trp Tyr Ser Tyr Tyr Asp Gly Val Thr Val
65                  70                  75                  80

Tyr Ser Pro Ser Glu Ile Asp Ile Asp His Val Val Pro Leu Ala Glu
                85                  90                  95

Ala Trp Arg Ser Gly Ala Ser Ser Trp Thr Thr Glu Lys Arg Gln Asn
            100                 105                 110

Phe Ala Asn Asp Leu Asn Gly Pro Gln Leu Ile Ala Val Thr Ala Ser
        115                 120                 125

Ser Asn Arg Ser Lys Gly Asp Gln Asp Pro Ser Thr Trp Gln Pro Thr
    130                 135                 140

Arg Thr Gly Ala Arg Cys Ala Tyr Ala Lys Met Trp Ile Asn Thr Lys
145                 150                 155                 160

Tyr Arg Trp Gly Leu His Leu Gln Ser Ser Glu Lys Ser Ala Leu Gln
                165                 170                 175

Ser Met Leu Asn Thr Cys Ser Tyr
            180

<210>  23
<211>  182
<212>  PRT
<213>  Bacillus marisflavi

<400>  23

Thr Pro Pro Val Thr Pro Ser Lys Glu Thr Ser Gln Ser Gln Leu Asn
1               5                   10                  15

Gly Leu Thr Val Lys Thr Glu Gly Ala Met Thr Gly Tyr Ser Arg Asp
            20                  25                  30

Lys Phe Pro His Trp Ser Ser Gln Gly Gly Gly Cys Asp Thr Arg Gln
        35                  40                  45

Val Val Leu Lys Arg Asp Ala Asp Ser Tyr Ser Gly Asn Cys Pro Val
    50                  55                  60

Thr Ser Gly Ser Trp Tyr Ser Tyr Tyr Asp Gly Val Lys Phe Thr His
65                  70                  75                  80

Pro Ser Asp Leu Asp Ile Asp His Ile Val Pro Leu Ala Glu Ala Trp
                85                  90                  95

Arg Ser Gly Ala Ser Ser Trp Thr Thr Ala Gln Arg Glu Ala Phe Ala
            100                 105                 110

Asn Asp Leu Ser Gly Ser Gln Leu Ile Ala Val Ser Ala Ser Ser Asn
        115                 120                 125

Arg Ser Lys Gly Asp Gln Asp Pro Ser Thr Trp Gln Pro Pro Arg Ala
    130                 135                 140

Gly Ala Lys Cys Gly Tyr Ala Lys Trp Trp Ile Ser Thr Lys Ser Lys
145                 150                 155                 160

Trp Asn Leu Ser Leu Gln Ser Ser Glu Lys Thr Ala Leu Gln Gly Met
                165                 170                 175

Leu Asn Ser Cys Val Tyr
            180

<210>  24
<211>  182
<212>  PRT
<213>  Bacillus sp. SA2-6

<400>  24

Leu Pro Ser Gly Ile Pro Ser Lys Ser Thr Ala Gln Ser Gln Leu Asn
1               5                   10                  15

Ser Leu Thr Val Lys Ser Glu Gly Ser Met Thr Gly Tyr Ser Arg Asp
            20                  25                  30

Lys Phe Pro His Trp Ile Ser Gln Gly Gly Gly Cys Asp Thr Arg Gln
        35                  40                  45

Val Val Leu Lys Arg Asp Ala Asp Tyr Tyr Ser Gly Asn Cys Pro Val
    50                  55                  60

Thr Ser Gly Lys Trp Tyr Ser Tyr Tyr Asp Gly Ile Ser Val Tyr Ser
65                  70                  75                  80

Pro Ser Glu Ile Asp Ile Asp His Val Val Pro Leu Ala Glu Ala Trp
                85                  90                  95

Arg Ser Gly Ala Ser Ser Trp Thr Thr Thr Lys Arg Gln Asn Phe Ala
            100                 105                 110

Asn Asp Leu Asn Gly Pro Gln Leu Ile Ala Val Thr Ala Ser Val Asn
        115                 120                 125

Arg Ser Lys Gly Asp Gln Asp Pro Ser Thr Trp Gln Pro Pro Arg Tyr
    130                 135                 140

Gly Ala Arg Cys Ala Tyr Ala Lys Met Trp Ile Asn Thr Lys Tyr Arg
145                 150                 155                 160

Trp Asp Leu Asn Leu Gln Ser Ser Glu Lys Ser Ser Leu Gln Ser Met
                165                 170                 175

Leu Asp Thr Cys Ser Tyr
            180

<210>  25
<211>  7
<212>  PRT
<213>  Artificial

<220>
<223>  Motif

<220>
<221>  MISC_FEATURE
<222>  (1)..(1)
<223>  Xaa = Asp (D) or Met (M) or Leu (L)

<220>
<221>  MISC_FEATURE
<222>  (2)..(2)
<223>  Xaa = Ser (S) or Thr (T)

<220>
<221>  MISC_FEATURE
<222>  (7)..(7)
<223>  Xaa = Asp (D) or Asn (N)

<400>  25

Xaa Xaa Gly Tyr Ser Arg Xaa
1               5

<210>  26
<211>  8
<212>  PRT
<213>  Artificial

<220>
<223>  Motif

<220>
<221>  MISC_FEATURE
<222>  (3)..(3)
<223>  Xaa = any amino acid

<400>  26

Ala Ser Xaa Asn Arg Ser Lys Gly
1               5

<210>  27

<211>  182
<212>  PRT
<213>  Bacillus cibi

<400>  27


<210>  28
<211>  269
<212>  PRT
<213>  Artificial Sequence

<220>
<223>  Variant

<400>  28

Ala Gln Ser Val Pro Trp Gly Ile Glu Arg Val Gln Ala Pro Ala Ala
1               5                   10                  15

His Asn Arg Gly Leu Thr Gly Ser Gly Val Lys Val Ala Val Leu Asp
            20                  25                  30

Thr Gly Ile Ser Thr His Pro Asp Leu Arg Ile Arg Gly Gly Ala Ser
        35                  40                  45

Phe Val Pro Gly Glu Pro Ser Thr Gln Asp Gly Asn Gly His Gly Thr
    50                  55                  60

His Val Ala Gly Thr Ile Ala Ala Leu Asp Asn Ser Ile Gly Val Leu
65                  70                  75                  80

Gly Val Ala Pro Ser Ala Glu Leu Tyr Ala Val Lys Val Leu Gly Ala
                85                  90                  95

Ser Gly Ser Gly Ser Val Ser Ser Ile Ala Gln Gly Leu Glu Trp Ala
            100                 105                 110

Gly Asn Asn Gly Met His Val Ala Asn Leu Ser Leu Gly Ser Pro Ser
        115                 120                 125

Pro Ser Ala Thr Leu Glu Gln Ala Val Asn Ser Ala Thr Ser Arg Gly
    130                 135                 140

Val Leu Val Val Ala Ala Ser Gly Asn Ser Gly Glu Pro Glu Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Pro Gly Leu Asp Ile
            180                 185                 190

Val Ala Pro Gly Val Asp Ile Leu Ser Thr Trp Pro Gly Gly Thr Tyr
        195                 200                 205

Ala Val Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Glu
                245                 250                 255

Tyr Gly Ser Gly Leu Val Asn Ala Glu Ala Ala Thr Arg
            260                 265